# EUROPEAN PATENT SPECIFICATION

(11) **EP 4 456 713 B1**
(45) Date of publication and mention of the grant of the patent: **12.11.2025**
(21) Application number: 22856986.9
(22) Date of filing: 29.12.2022
(51) Int. Cl.: A01H 3/00, A01H 4/00, C12N 1/20, C12R 1/01

(54) **PRODUCTS AND METHODS FOR IMPROVING PLANT GROWTH FEATURES**
PRODUKTE UND VERFAHREN ZUR VERBESSERUNG VON PFLANZENWACHSTUMSMERKMALEN
PRODUITS ET PROCÉDÉS POUR AMÉLIORER LES CARACTÉRISTIQUES DE CROISSANCE VÉGÉTALE

(30) Priority: 30.12.2021 EP 21218360
(43) Date of publication of application: 06.11.2024
(73) Proprietor: Aphea.Bio NV, 9052 Zwijnaarde (BE)
(72) Inventor: VIAENE, Tom, 9000 Gent (BE); HAMONTS, Kelly, 9000 Gent (BE); GHODSALAVI, Behnoush, 9000 Gent (BE); IMPERATO, Valeria, 9000 Gent (BE); VANDENABEELE, Steven, 9700 Oudenaarde (BE); VERCAUTEREN, Isabel, 9550 Woubrechtegem (BE)
(74) Representative: De Clercq & Partners
(86) International application number: PCT/EP2022/087982
(87) International publication number: WO 2023/126460

(56) References cited:
- WO-A1-2020/214843
- WO-A1-2021/012000
- WO-A2-2017/019633
- WO-A2-2021/258073
- CN-A- 111 533 585
- DATABASE Geneseq [online] 10 December 2020 (2020-12-10), FUENZALIDA G.: "Stenotrophomonas rhizophila 16S polynucleotide SEQ: 3464.", XP055932984, retrieved from EBI Database accession no. BIM19112
- DATABASE Geneseq [online] 16 June 2022 (2022-06-16), BALLOK A.: "Stenotrophomonas rhizophila 16S rRNA sequence, SEQ ID 19.", XP055932992, retrieved from EBI Database accession no. BKM20175
- MAJEED AFSHAN ET AL: "Isolation and characterization of plant growth-promoting rhizobacteria from wheat rhizosphere and their effect on plant growth promotion", FRONTIERS IN MICROBIOLOGY, vol. 6, 17 March 2015 (2015-03-17), XP055933129, DOI: 10.3389/fmicb.2015.00198
- TABACCHIONI SILVIA ET AL: "Identification of Beneficial Microbial Consortia and Bioactive Compounds with Potential as Plant Biostimulants for a Sustainable Agriculture", MICROORGANISMS, vol. 9, no. 2, 19 February 2021 (2021-02-19), pages 426, XP055929659, DOI: 10.3390/microorganisms9020426

## Description

### FIELD OF THE INVENTION

The invention is broadly in the field of plant biology and bacterial strains, more precisely in the field of agricultural biologicals or agro-biologicals. In particular, the invention relates to products and methods for enhancing certain plant growth characteristics, and to a novel bacterial strain useful as a biostimulant.

### BACKGROUND OF THE INVENTION

There is a need for improved agricultural plants that will enable the food production demands with fewer resources and more environmentally sustainable inputs, for plants with improved responses to various biotic and abiotic stresses.

Crop performance is optimized primarily via technologies directed towards the interplay between crop genotype (e.g. plant breeding, genetically-modified (GM) crops) and its surrounding environment (e.g. fertilizer, synthetic herbicides, pesticides). While these paradigms have assisted in the increasing global food production, yield growth rates have stalled in many major crops. Shifts in the climate are linked to production instabilities as well as changing pest and disease pressures. In addition, genetically manipulated (GM) crops and agrochemicals have been challenged in their use in a large number of agricultural important crops and countries, resulting in a lack of acceptance for many GM traits and the exclusion of GM crops and many agrochemicals from global markets. Therefore, there is an urgent need for novel solutions to crop improvement, more particularly, there is a need for innovative, effective, environmentally-sustainable, and publicly-acceptable approaches to improve the biomass, yield, and other agronomically important characteristics of plants.

A promising practice is the use of microorganisms that enhance plant growth and yield, increase tolerance to unfavorable conditions, and/or improve the resource use efficiency. In particular, a vast array of bacteria that live both within and around the plant tissues support the plant's health and growth.

Bacteria influence plant growth through multiple mechanisms, and in some cases through interactions with other bacteria. Specific bacterial strains inhabit various host plant tissues and have been isolated from plant leaves, stems, and roots. Several bacteria have been disclosed that increase plant growth and/or reduce susceptibility to diseases caused by fungi, bacteria, viruses or other plant pathogens.

The present invention aims to address at least some of the circumstances mentioned above. The aim of the invention is to provide further and/or improved means and methods to enhance agriculturally useful characteristics of an agricultural plant.

### SUMMARY OF THE INVENTION

The present disclosure is at least in part based on the discovery that certain bacteria can be used as an agricultural biological, in particular as a biostimulant, to increase one or more plant trait of agronomic importance, such as in particular the biomass, height, and/or yield of a plant or part thereof.

The bacterial strains, products, methods, and uses herein disclosed advantageously allow to improve one or more trait of agronomic importance in a plant, such as one or more plant growth features. Hence, the herein described bacterial strains provide several significant advantages to plants, in particular to agricultural plants, such as wheat, barley, maize, and the like. For example, dry biomass, wet biomass, number of tillers per plant, height of the plant, plant yield, seed yield, fruit yield, and/or emergence of a plant can be increased compared to untreated plants by applying the teachings of the present disclosure. Further the herein described bacterial strain also improves the ability of the plants to cope with abiotic stresses such as, but not limited to, drought. The present disclosure can thus allow to substitute or even abolish the use of chemical products such as fertilizers, and thereby advantageously facilitate more sustainable agriculture or increase the yield under adverse conditions. The teachings of the present disclosure can be immediately applied to any plant and, compared to provision of transgenic plants, do not require additional time for gene identification, generation and characterization of transgenic lines. Compared to the use of traditional agricultural methods including the application of chemical fertilizers, the present approaches can require less resources, can be less labor intensive, and are more environmentally friendly, and thereby also compatible with organic farming practices.

The present invention provides subject-matter as set forth in any one of all of the appended claims.

The above and further aspects and preferred examples of the disclosure are described in the following sections and in the appended claims. The subject-matter of appended claims is hereby specifically incorporated in this specification.

### DESCRIPTION OF THE DRAWINGS

The following description of the figures of specific examples of the disclosure is merely exemplary in nature and is not intended to limit the present teachings, their application or uses.

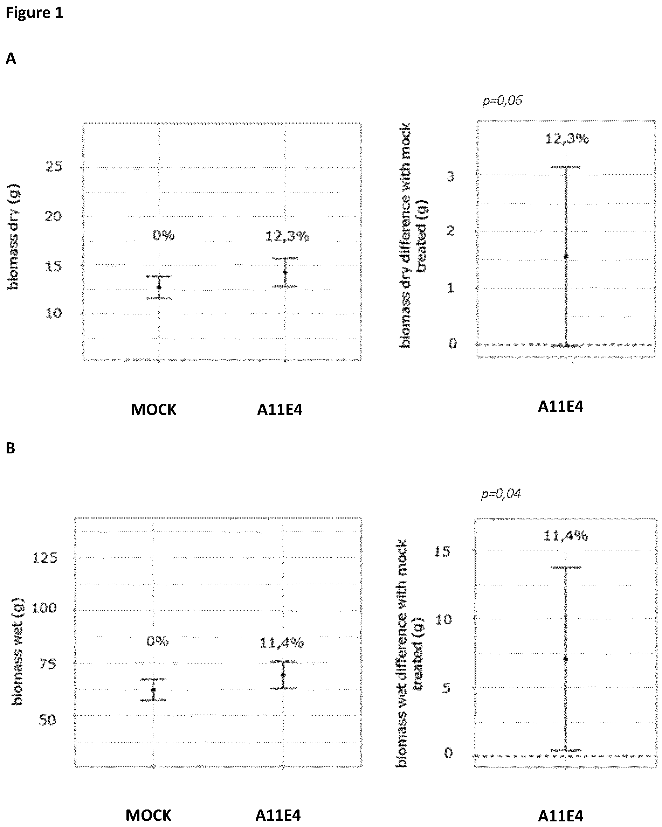

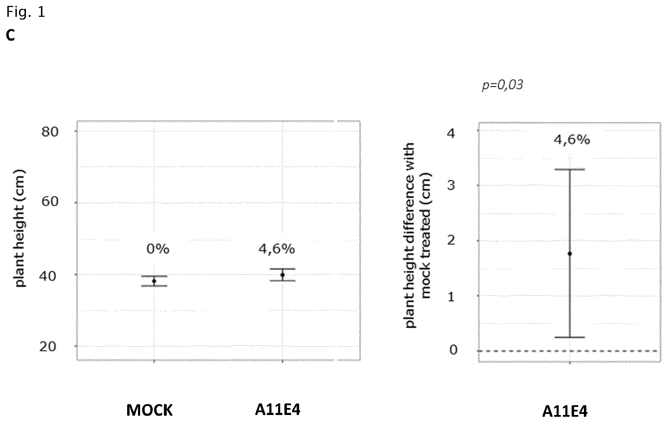

In **Figure 1****,** the graphs on the left visualize the values of the dry biomass, wet biomass, or plant height per plant, with 95% confidence intervals for treated seeds and mock treated seeds in greenhouse condition, whereas the graphs on the right visualize the values of the difference between treated and mock treated seeds in dry biomass, wet biomass, or plant height per plant with its 95% confidence interval in greenhouse condition. The percentage indicates the difference in dry biomass, wet biomass, or plant height per plant expressed as a percentage of the mock treatment.
**Figure 1A** represents graphs illustrating the increased dry biomass per plant at 6 weeks after sowing of wheat plants obtained from seeds treated with a formulation comprising the A11E4 bacterial strain compared to the dry biomass per plant at 6 weeks after sowing of wheat plants obtained from untreated seeds (mock).
**Figure 1B** represents graphs illustrating the increased wet biomass per plant at 6 weeks after sowing of wheat plants obtained from seeds treated with a formulation comprising the A11E4 bacterial strain compared to the wet biomass per plant at 6 weeks after sowing of wheat plants obtained from untreated seeds (mock).
**Figure 1C** represents graphs illustrating the increased plant height per plant at 6 weeks after sowing of wheat plants obtained from seeds treated with a formulation comprising the A11E4 bacterial strain compared to the plant height per plant at 6 weeks after sowing of wheat plants obtained from untreated seeds (mock).

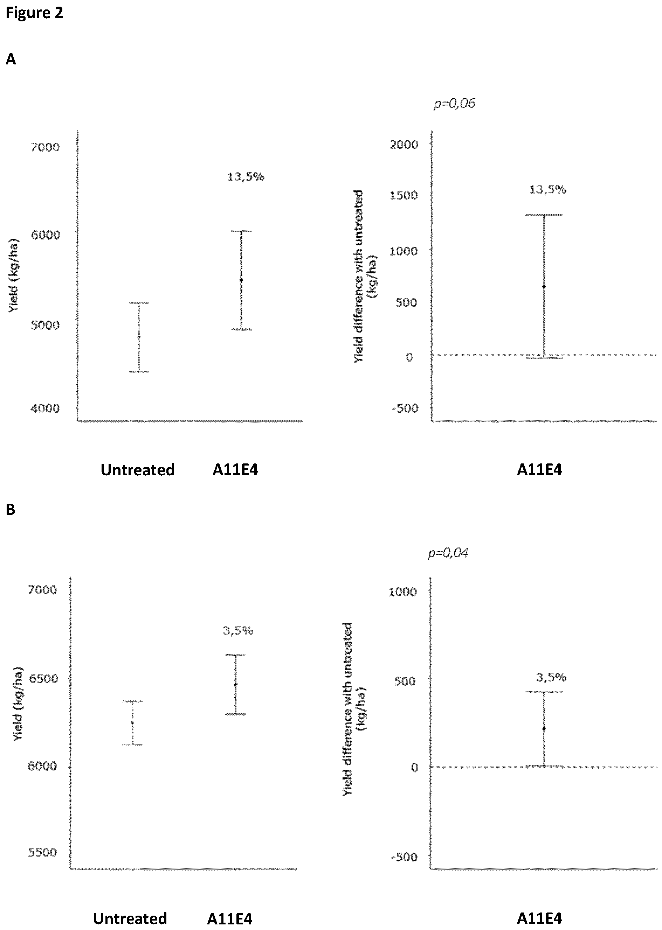

In **Figure 2****,** the graph on the left visualizes the values of spring wheat grain yield with 95% confidence intervals for treated seeds and untreated seeds, whereas the graph on the right visualizes the values of the difference between treated and untreated seeds in grain yield with its 95% confidence interval. The percentage indicates the difference in wheat grain yield expressed as a percentage of the mock treatment.
**Figure 2A** represents graphs illustrating the increased seed yield obtained from spring wheat seeds grown at one individual location. The wheat plants were obtained from wheat seeds treated with a formulation comprising the A11E4 bacterial strain and compared to wheat plants obtained from untreated wheat seeds (dashed line).
**Figure 2B** represents graphs illustrating the increased seed yield obtained from spring wheat seeds grown at one individual location. The wheat plants were obtained from wheat seeds treated with a formulation comprising the A11E4 bacterial strain and compared to wheat plants obtained from untreated wheat seeds (dashed line).

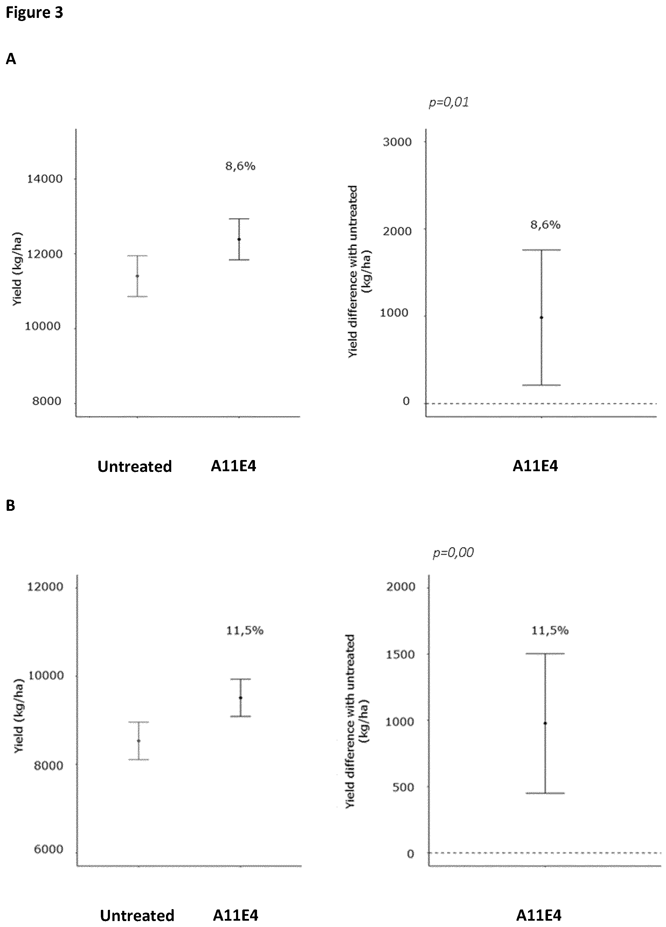

In **Figure 3** the graph on the left visualizes the values of winter wheat grain yield with 95% confidence intervals for treated seeds and untreated seeds, whereas the graph on the right visualizes the values of the difference between treated and untreated seeds in grain yield with its 95% confidence interval. The percentage indicates the difference in wheat grain yield expressed as a percentage of the untreated seeds.
**Figure 3A** represents graphs illustrating the increased seed yield obtained from wheat seeds grown at one individual location. The wheat plants were obtained from wheat seeds treated with a formulation comprising the A11E4 bacterial strain and compared to wheat plants obtained from untreated wheat seeds (dashed line).
**Figure 3B** represents graphs illustrating the increased seed yield obtained from wheat seeds grown at one individual location. The wheat plants were obtained from wheat seeds treated with a formulation comprising the A11E4 bacterial strain and compared to wheat plants obtained from untreated wheat seeds (dashed line).

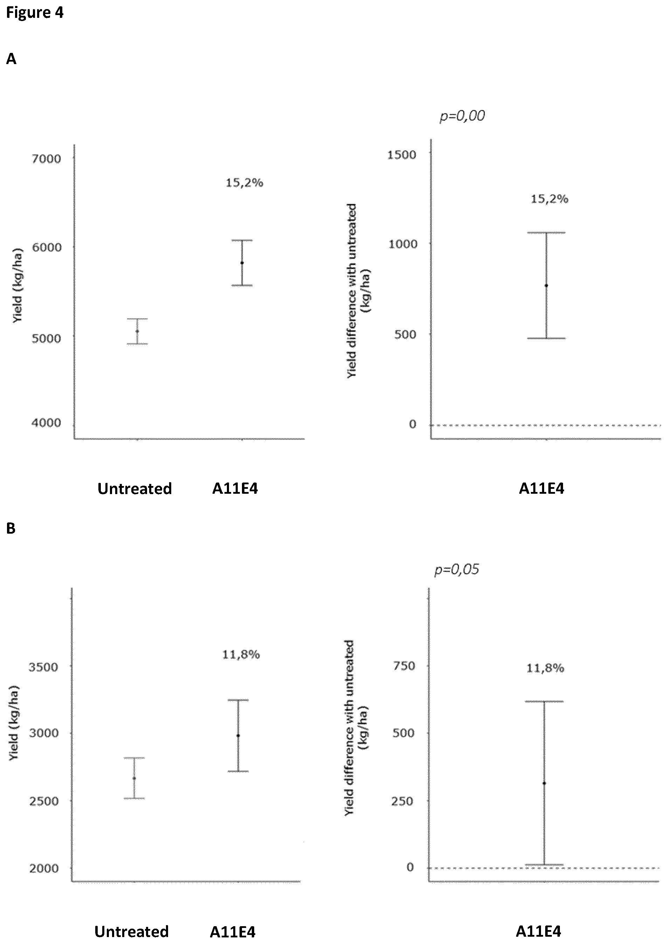

In **Figure 4** the graph on the left visualizes the values of winter wheat grain yield with 95% confidence intervals for treated seeds and untreated seeds, whereas the graph on the right visualizes the values of the difference between treated and untreated seeds in grain yield with its 95% confidence interval. The percentage indicates the difference in wheat grain yield expressed as a percentage of the untreated seeds. Fig. 3 and Fig. 4 both relate to winter wheat grain yield, and show experiments from different seasons (winter 2020 and winter 2021).
**Figure 4A** represents graphs illustrating the increased seed yield obtained from wheat seeds grown at one individual location. The wheat plants were obtained from wheat seeds treated with a formulation comprising the A11E4 bacterial strain and compared to wheat plants obtained from untreated wheat seeds (dashed line).
**Figure 4B** represents graphs illustrating the increased seed yield obtained from wheat seeds grown at one individual location. The wheat plants were obtained from wheat seeds treated with a formulation comprising the A11E4 bacterial strain and compared to wheat plants obtained from untreated wheat seeds (dashed line).

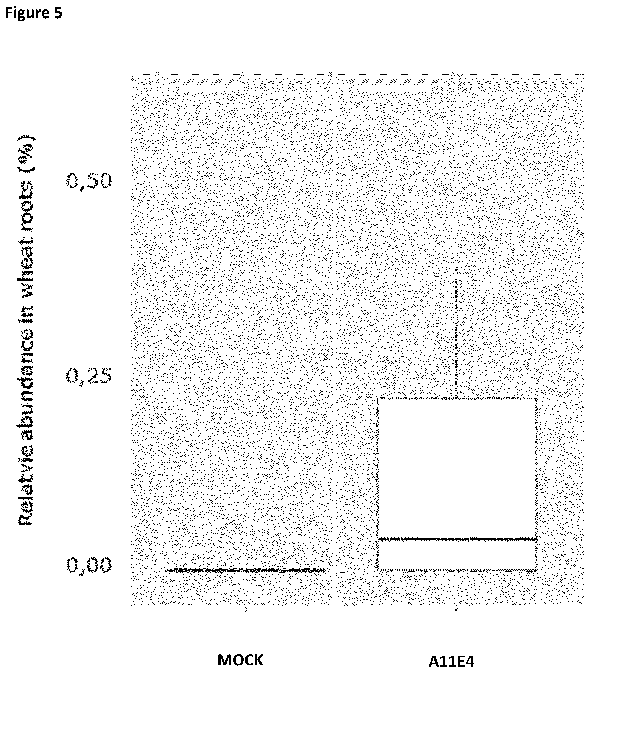

**Figure 5** shows the relative abundance of the amplicon of the A11E4 strain present in the wheat roots and is a graphical representation of wheat root colonization in the field. Roots were obtained from plants grown from wheat seeds treated with a formulation comprising the A11E4 bacterial strain.

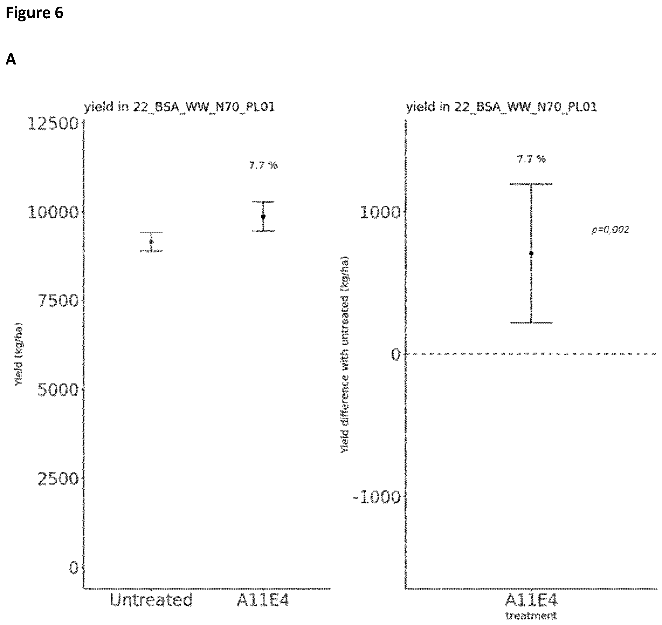

**Figure 6A** visualizes the value in grain yield of winter wheat measured at a location in Poland in the season 2021-2022 with a 70 % N fertilizer regime. The graph on the left visualizes the values of winter wheat grain yield with 95% confidence intervals for treated seeds and untreated seeds, whereas the graph on the right visualizes the values of the difference between treated and untreated seeds in grain yield with its 95% confidence interval. Wheat treated with the spray-dried formulation containing a *Stenotrophomonas A11E4 strain* and colorant showed an increased yield of 7.7 % compared to the untreated seeds.

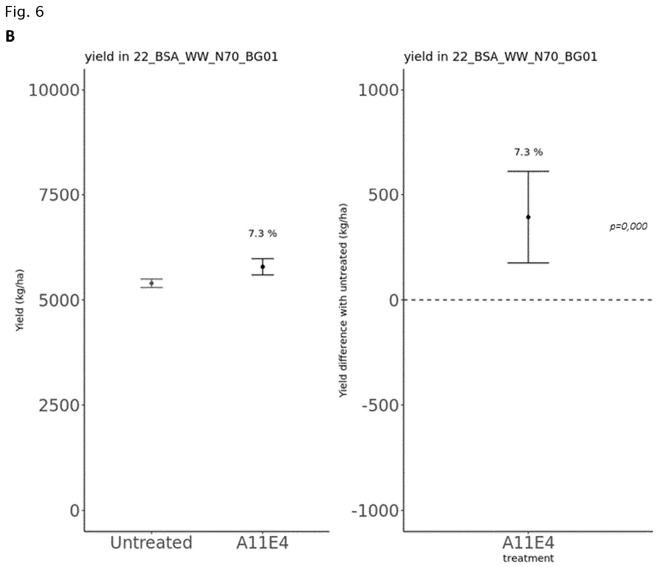

**Figure 6B** visualizes the value in grain yield of winter wheat measured at a location in Bulgaria in the season 2021-2022 with a 70 % N fertilizer regime. The graph on the left visualizes the values of winter wheat grain yield with 95% confidence intervals for treated seeds and untreated seeds, whereas the graph on the right visualizes the values of the difference between treated and untreated seeds in grain yield with its 95% confidence interval. Wheat treated with the spray-dried formulation containing a *Stenotrophomonas A11E4 strain* and colorant showed an increased yield of 7.3 % compared to the untreated seeds.

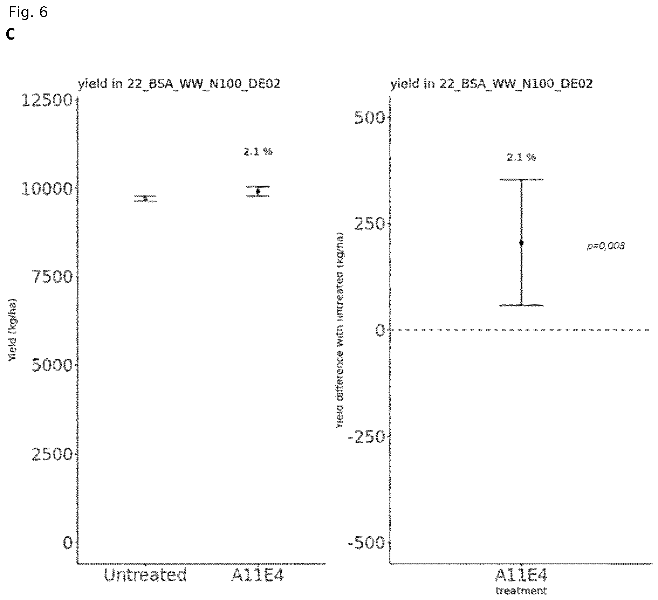

**Figure 6C** visualizes the value in grain yield of winter wheat measured at a location in Germany in the season 2021-2022 with a 100 % N fertilizer regime. The graph on the left visualizes the values of winter wheat grain yield with 95% confidence intervals for treated seeds and untreated seeds, whereas the graph on the right visualizes the values of the difference between treated and untreated seeds in grain yield with its 95% confidence interval. Wheat treated with the spray-dried formulation containing a *Stenotrophomonas A11E4 strain* and colorant showed an increased yield of 2.1 % compared to the untreated seeds.

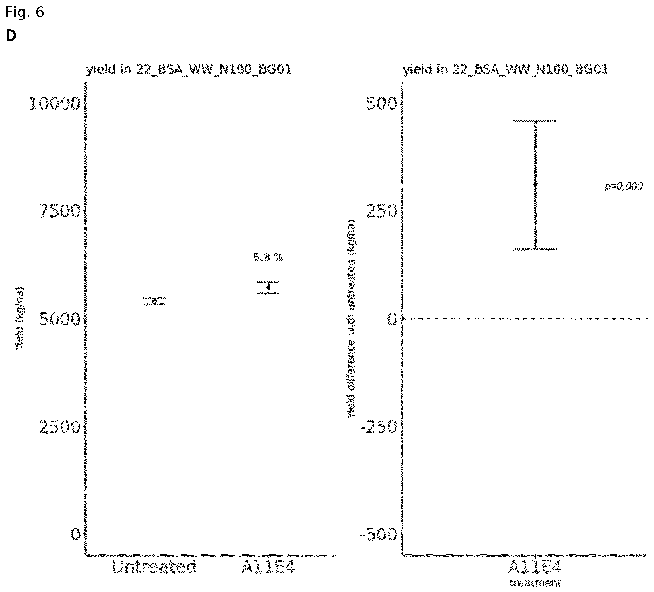

**Figure 6D** visualizes the value in grain yield of winter wheat measured at a location in Bulgaria in the season 2021-2022 with a 100 % N fertilizer regime. The graph on the left visualizes the values of winter wheat grain yield with 95% confidence intervals for treated seeds and untreated seeds, whereas the graph on the right visualizes the values of the difference between treated and untreated seeds in grain yield with its 95% confidence interval. Wheat treated with the spray-dried formulation containing a *Stenotrophomonas A11E4 strain* and colorant showed an increased yield of 5.8 % compared to the untreated seeds.

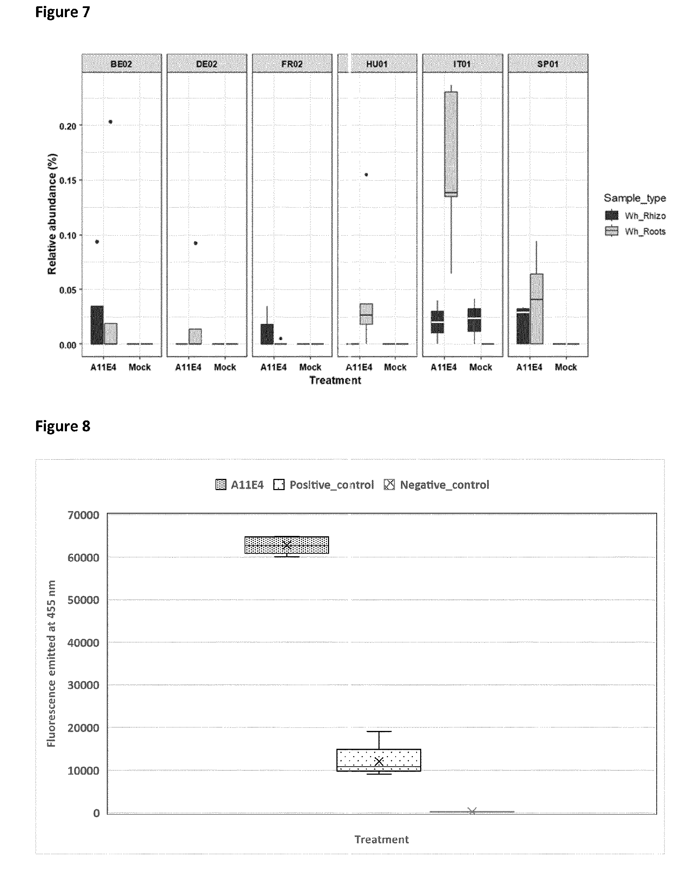

**Figure 7** shows relative abundance of the amplicon of the A11E4 strain present in the wheat rhizosphere soil (Wh_Rhizo) and wheat roots (Wh_Roots), as evidence for colonization of wheat plants in six field trials. Samples were obtained from plants grown from wheat seeds treated with a formulation comprising the A11E4 bacterial strain or from seeds treated with a colorant only (Mock). Plants were harvested at growth developmental stage BBCH16-17 in six field trials across Europe (BE02: in Belgium; DE02: in Germany; FR02: in France; HU01: in Hungary; IT01: in Italy; SP01: in Spain).

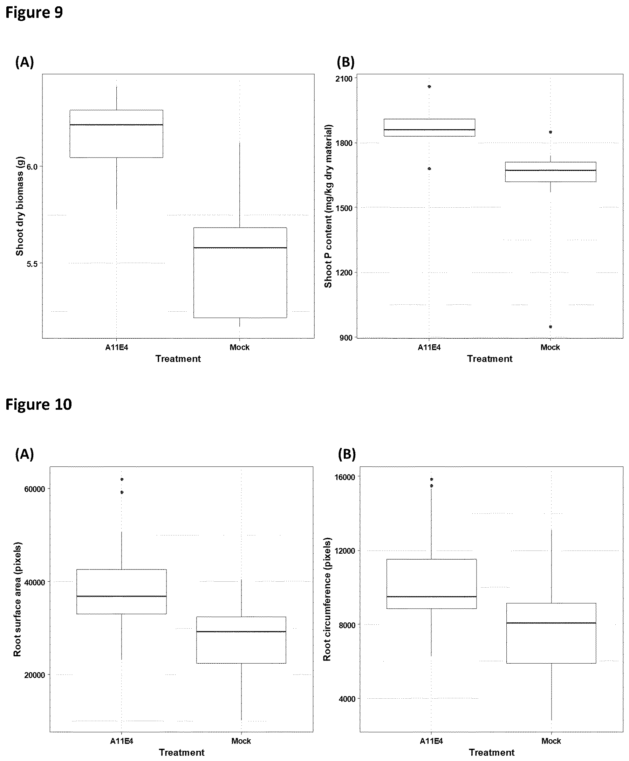

**Figure 8** shows phosphatase enzyme activity of strain A11E4 as determined by the EnzChek^{™} Phosphatases Assay Kit. The positive control corresponds to the phosphatase activity of DSM strain 17497.**Figure 9** shows shoot dry biomass **(A)** and shoot relative P content **(B)** of wheat plants treated with the A11E4 bacterial strain (treatment A11E4) or without (Mock treatment), harvested 6 weeks after sowing. Boxplots show data for five replicate planter boxes per treatment, each containing 24 wheat plants.
**Figure 10** shows root surface area **(A)** and root circumference **(B)** of wheat plants treated with the A11E4 bacterial strain (A11E4 treatment) or without (Mock treatment) after *in vitro* germination of the treated wheat seeds on soil extract medium agar plates and incubation in the dark. Roots were scanned seven days after inoculation. Boxplots represent 30 replicates per treatment.

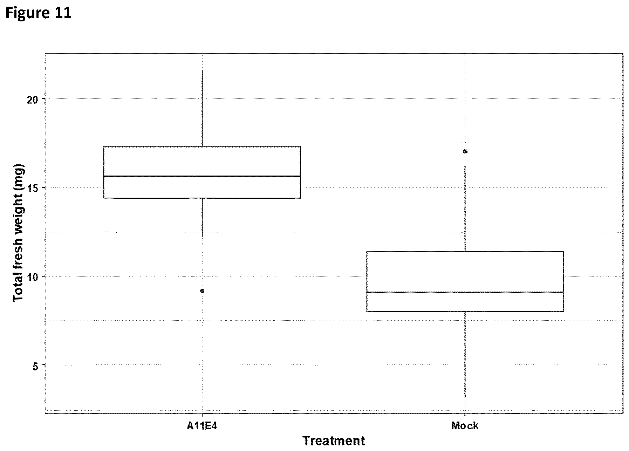

**Figure 11** shows fresh biomass results from *in vitro Arabidopsis thaliana* growth promotion assay, showing increased biomass for seedlings treated with bacterial strain A11E4 compared to the Mock.

### DETAILED DESCRIPTION

As used herein, the singular forms "a", "an", and "the" include both singular and plural referents unless the context clearly dictates otherwise.

The terms "comprise", "comprising", "comprises" and "comprised of" as used herein are synonymous with "include", "including", "includes" or "contain", "containing", "contains", and are inclusive or open-ended and do not exclude additional, non-recited members, elements or method steps. The terms also encompass "consisting of" and "consisting essentially of".

The recitation of numerical ranges by endpoints includes all numbers and fractions subsumed within the respective ranges, as well as the recited endpoints. This applies to numerical ranges irrespective of whether they are introduced by the expression "from... to..." or the expression "between... and..." or another expression.

The terms "about" or "approximately" as used herein when referring to a measurable value such as a parameter, an amount, a temporal duration, and the like, are meant to encompass variations of and from the specified value, such as variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" or "approximately" refers is itself also specifically, and preferably, disclosed.

Furthermore, the terms first, second, third and the like in the description and in the claims, are used for distinguishing between similar elements and not necessarily for describing a sequential or chronological order, unless specified. It is to be understood that the terms so used are interchangeable under appropriate circumstances and that the examples of the disclosure are capable of operation in other sequences than described or illustrated herein.

Whereas the term "one or more" or "at least one", such as one or more or at least one member(s) of a group of members, is clear *per se,* by means of further exemplification, the term encompasses *inter alia* a reference to any one of said members, or to any two or more of said members, such as, e.g., any ≥3, ≥4, ≥5, ≥6 or ≥7 etc. of said members, and up to all said members. In another example, "one or more" or "at least one" may refer to 1, 2, 3, 4, 5, 6, 7 or more.

The discussion of the background to the disclosure herein is included to explain the context of the disclosure. This is not to be taken as an admission that any of the material referred to was published, known, or part of the common general knowledge in any country as of the priority date of any of the claims.

Throughout this disclosure, various publications, patents and published patent specifications may be referenced by an identifying citation.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the invention. When specific terms are defined in connection with a particular aspect of the disclosure or a particular example of the disclosure, such connotation or meaning is meant to apply throughout this specification, i.e., also in the context of other aspects or examples of the disclosure, unless otherwise defined.

In the following passages, different aspects or examples of the disclosure are defined in more detail. Each aspect or example so defined may be combined with any other aspect(s) or example(s) unless clearly indicated to the contrary. In particular, any feature indicated as being preferred or advantageous may be combined with any other feature or features indicated as being preferred or advantageous.

Reference throughout this specification to "one example", "an example" means that a particular feature, structure or characteristic described in connection with the example is included in at least one example of the present disclosure. Thus, appearances of the phrases "in one example" or "in an example" in various places throughout this specification are not necessarily all referring to the same example, but may. Furthermore, the particular features, structures or characteristics may be combined in any suitable manner, as would be apparent to a person skilled in the art from this disclosure, in one or more examples.

By extensive experimental testing, the present inventors have found that certain bacterial strains exhibit plant growth promoting effects when administered to plants and hence that such strains can advantageously be used as biostimulants on plants. In particular, the bacterial strains provided an unexpected enhancement of plant biomass, height, and/or yield, such as wet and dry biomass, height of plants, and seed yield, in tested plants.

Accordingly, a method is disclosed for improving a plant growth feature of a plant compared to an untreated plant, the method comprising administering bacteria of a bacterial strain which comprises a 16S polynucleotide having at least 99.75% sequence identity to SEQ ID NO: 1 to the plant, a part thereof, a seed for growing the plant, or a locus of the plant. For example, such methods may be suitably practiced in the context of agriculture or horticulture.

Also disclosed is a method of treating a seed of a plant comprising inoculating the seed with said bacteria, such that the bacteria colonize a plant germinated from the inoculated seed and/or the soil or plant growth medium surrounding the growing plant, whereby the plant growth feature of the plant is improved compared to a plant germinated from an untreated seed.

A plant or part thereof treated with said bacteria or with a composition comprising the bacteria; or a plant or part thereof heterologously disposed with said bacteria, are also described herein.

A further aspect discloses a bacterial strain as deposited under the Budapest Treaty at the BCCM^{™} / LMG Bacteria collection (BCCM^{™}/LMG) on 24 December 2021 under Accession No. LMG P-32513 (with the following identification reference given to the deposited material by the depositor: **BML-B-A11E4(2)**), or a functional mutant thereof. The proposed taxonomic designation of this strain is *Stenotrophomonas rhizophila.* Also described is a bacterial population comprising the aforementioned strain, as well as an agricultural active composition comprising the aforementioned strain.

As used herein, the term "bacterium", "bacteria", or "bacterial" refers in general to any prokaryotic organism, and may refer to an organism from either Kingdom Eubacteria (Bacteria), Kingdom Archaebacteria (Archaea), or both. In some cases, bacterial genera have been reassigned due to various reasons (such as, but not limited to, the evolving field of whole genome sequencing), and it is understood that such nomenclature reassignments are within the scope of any claimed genus.

As used herein, "bacterial strain" (which may be abridged to "strain" where the context makes clear that a bacterial strain is meant) refers to any of the prokaryotic microorganism belonging to the same class of species, including the species. The term "strain" as a basic operational unit of microbial taxonomy, such as bacterial taxonomy, is frequently used to denote a population made up of the descendants of a single isolation in pure culture, usually made up of a succession of cultures ultimately derived from an initial single colony. Where a species encompasses two or more distinct isolates, the term "strain" may be used to refer to an isolate or group of isolates that can be distinguished from other isolates of the same genus and species by phenotypic characteristics or genotypic characteristics or both.

In the practice of the present disclosure, the strain may be deemed as "isolated" or "purified". The terms "isolated" or "purified" with reference to a particular component generally denote that such component exists in separation from - for example, has been separated from or prepared and/or maintained in separation from - one or more other components of its natural environment. The terms do not necessarily reflect the extent to which the component has been purified. Hence, the phrases "isolated bacterial strain" or "purified bacterial strain" may be seen as referring to a strain that has been removed from its natural milieu. In particular, the terms refer to substantially no other strains than the desired strain, which is thus substantially free of other contaminants, which can include microbial contaminants. Further, the terms may denote that the strain has been separated from materials with which it is normally found in nature. A strain heterologously disposed to other strains, or with compounds or materials with which it is not normally found in nature, is encompassed by the phrases "isolated bacterial strain" or "purified bacterial strain".

In certain examples, the purified bacterial strains as taught herein may be denoted as endophytes. An "endophyte" is an organism capable of living on a plant element (e.g., rhizoplane or phyllosphere) or within a plant element (e.g., endosphere) or on a surface in close physical proximity with a plant element (e.g., the rhizosphere or on a seed). Endophytes can occupy the intracellular or extracellular spaces of plant tissue, including but not limited to leaves, stems, flowers, fruits, seeds, or roots. An endophyte can be, for example, a bacterial or fungal organism, and can confer a beneficial property to the host plant such as an increase in yield, biomass, resistance, and/or fitness. An endophyte can be a bacterium or a fungus. As used herein, the term "microbe" or "strain" is sometimes used to describe an endophyte. As used herein, the microbes or strains as described herein can be labelled as endophytes.

Endophytes may favorably impact one or more traits of agronomic interest in plants. By means of an example and without limitation, a plant heterologously disposed with one or more endophyte microorganism, or a plant grown from a plant part or seed treated with or heterologously disposed with one or more endophyte microorganism, such as an endophytic bacterial or fungal strain, may exhibit a trait of agronomic interest, such as a trait selected from the group consisting of: disease resistance, drought tolerance, heat tolerance, cold tolerance, salinity tolerance, metal tolerance, herbicide tolerance, chemical tolerance, improved water use efficiency, improved phosphorus solubilization, improved phosphorus mobilization, improved nitrogen utilization, improved nitrogen fixation, pest resistance, herbivore resistance, pathogen resistance, increase in yield, increase in yield under water-limited conditions, health enhancement, vigor improvement, growth improvement, improved plant emergence, photosynthetic capability improvement, nutrition enhancement, altered protein content, altered oil content, increase in biomass, increase in number of tillers per plant, increase in shoot length, increase in root length, improved root architecture, increase in seed weight, altered seed carbohydrate composition, altered seed oil composition, increase in radical length, delayed senescence, stay-green, altered seed protein composition, increase in dry weight of mature plant reproductive elements, increase in fresh weight of mature plant reproductive elements, increase in number of mature plant reproductive elements per plant, increase in chlorophyll content, reduced number of wilted leaves per plant, reduced number of severely wilted leaves per plant, increase in number of non-wilted leaves per plant, improved plant visual appearance, and combinations thereof.

As used herein, a microorganism, such as a bacterial or fungal strain, such as an endophytic bacterial or fungal strain, is considered to have conferred an improved agricultural trait whether or not the improved trait arose from the plant, the strain, or the concerted action between the plant and the strain. Therefore, for example, where an improved agronomic trait results at least in part from the production of a beneficial hormone or chemical, for the purposes of the present specification the strain will be considered to have conferred the improved agronomic trait upon the plant as compared to a plant, plant part or seed that has not been treated with or heterologously disposed with said strain, whether the beneficial hormone or chemical is produced by the plant or by the strain.

Particularly envisaged herein is the administration of live bacteria and/or microorganisms. The term "live" as used herein is synonymous with "viable" and refers to any living intact state of a microorganism, such as active growth or dormancy, from which state it can multiply and/or reproduce itself in a medium capable of supporting the growth of the microorganism. Typically, substantially all bacteria or microorganisms comprised by populations or compositions intended herein may be live or viable. For example, at least 50%, preferably at least 60%, more preferably at least 75%, still more preferably at least 90%, such as at least 95%, 96%, 97%, 98%, 99% or 100% of the bacteria or microorganisms in the population or composition may be viable, such as capable of forming colonies when plated on a suitable solid medium.

The term "16S polynucleotide", or synonymous terms such as "16S nucleotide sequence" or "16S", refer to the nucleic acid sequence, such as in particular the DNA sequence, of the 16S ribosomal RNA (rRNA) of a bacterium. 16S rRNA gene sequencing is a well-established method for studying phylogeny and taxonomy of bacteria. A full length 16S nucleic acid sequence is approximately 1500 nucleotides in length. The bacterial strain may comprise a single copy of the 16S rRNA gene. The bacterial strain may comprise more than one copy of the 16S rRNA gene, such as two, three or more (multicopy) copies of the 16S rRNA gene. Where two or more 16S rRNA gene copies are found in the bacterial strain, at least one of these 16S rRNA gene copies complies with the sequence identity requirements specified in the present disclosure, preferably two or more and preferably all of the 16S rRNA gene copies (each independently) comply with the stated sequence identity requirements. By means of an example and without limitation, where a bacterial strain comprises three 16S rRNA gene copies, at least one, preferably at least two, and more preferably all three 16S rRNA gene copies will, each independently, display at least 99.75% sequence identity to SEQ ID NO: 1, and may in certain preferred examples be identical. Conveniently, the 16S rRNA sequence can be determined by sequencing (e.g., Sanger sequencing) the 16S gene sequence(s) in the chromosomal DNA, which may be amplified (e.g., PCR amplified) using suitable amplification primers, such as in particular the Forward primer 27F: AGAGTTTGATCCTGGCTCAG (SEQ ID NO: 2) and Reverse primer1492R: GGTTACCTTGTTACGACTT (SEQ ID NO: 3).

The terms "identity", "sequence identity" or "identical" in the context of nucleotide sequences may be used interchangeably herein, and refer to the extent that nucleic acid sequences are identical on a nucleotide-by-nucleotide basis, over a window of comparison. The percentage of sequence identity may be calculated by comparing two optimally aligned sequences over the window of comparison, determining the number of positions at which the identical nucleic acid base occurs in both sequences to yield the number of matched positions, dividing the number of matched positions by the total number of positions in the window of comparison, and multiplying the result by 100 to yield the percentage of sequence identity. The percent identity value may, but need not, be rounded to the nearest tenth. For example, 98.11, 98.12, 98.13, and 98.14 may be rounded down to 98.1, while 98.15, 98.16, 98.17, 98.18, and 98.19 may be rounded up to 98.2.

Sequence identity between nucleic acids as envisaged herein may be determined using suitable algorithms for performing sequence alignments and determination of sequence identity as know *per se.* Exemplary but non-limiting algorithms include those based on the Basic Local Alignment Search Tool (BLAST) originally described by Altschul et al. 1990 (J Mol Biol 215: 403-10), such as the "Blast 2 sequences" tool described by Tatusova and Madden 1999 (FEMS Microbiol Lett 174: 247-250), or the "blastn suite-2sequences" sequence alignment algorithm described by Zheng Zhang et al. 2000 (J Comput Biol 2000, vol. 7(1-2), 203-14), now incorporated into the BLAST program suite available at ncbi.nlm.nih.gov. The skilled person can implement such algorithms and set the requisite parameters. By means of an example and without limitation, parameters for the BLASTN program may be as follows: cost to open a gap = 0, cost to extend a gap = 2.5, reward for a match = 1, penalty for a mismatch = -2, Expect value = 0.05, word size = 28, Low Complexity Filter = Yes.

There are further algorithms known in the art that can be used to measure nucleotide sequence identity. Nucleotide sequence identity can be measured by a local or global alignment, preferably implementing an optimal local or optimal global alignment algorithm. For example, a global alignment may be generated using an implementation of the Needleman-Wunsch algorithm (Needleman & Wunsch. Journal of Molecular Biology 1970, vol. 48(3), 443-53). For example, a local alignment (which does not consider the entirety of the sequence but tries to find the longest subsequence that confirms to a given matching criteria) may be generated using an implementation of the Smith-Waterman algorithm (Smith & Waterman Journal of Molecular Biology 1981, vol. 147(1), 195-197). Optimal global alignments using the Needleman-Wunsch algorithm and optimal local alignments using the Smith-Waterman algorithm are implemented in USEARCH (https://www.drive5.com/usearch/), for example USEARCH version 11.0.667.

A gap is a region of an alignment wherein a sequence does not align to a position in the other sequence of the alignment. In global alignments, terminal gaps are discarded before identity is calculated. For both local and global alignments, internal gaps are counted as differences. A terminal gap is a region beginning at the end of a sequence in an alignment wherein the nucleotide in the terminal position of that sequence does not correspond to a nucleotide position in the other sequence of the alignment and extending for all contiguous positions in that sequence wherein the nucleotides of that sequence do not correspond to a nucleotide position in the other sequence of the alignment.

Sequence identity as envisaged herein in particular denotes overall sequence identity, i.e., sequence identity calculated from optimally aligning the whole sequences of the to-be-compared 16S rRNA genes. In other words, the nucleic acid sequences to be aligned are the complete 16S rRNA genes, and the window of comparison corresponds to the whole region of optimal alignment between these complete 16S sequences, i.e., to the alignment length. Hence, in an example, a query 16S rRNA gene sequence, such as the sequence set forth in SEQ ID NO: 1, is optimally aligned with another 16S rRNA gene sequence (in case of a global alignment any terminal gaps are disregarded; in case of a local alignment the region of alignment will be expressed as the region between a given 5' and a given 3' position in the query sequence), and the percentage sequence identity is calculated over a window of comparison which corresponds to the whole region of alignment, counting internal gaps as differences.

Bacterial strains as described herein preferably comprise a 16S polynucleotide the length of which is between 90% and 110% (1390-1698 nucleotides), more preferably between 95% and 105% (1467-1621 nucleotides) of the length of the 16S region polynucleotide shown in SEQ ID NO: 1. These 16S sequences can be subjected to pairwise sequence comparisons with SEQ ID NO: 1.

When two complete 16S region sequences in a pairwise sequence comparison are optimally aligned, for example by a local alignment algorithm such as BLAST, it is particularly envisaged that the alignment length is at least 90% of the length of the shorter one of the two 16S sequences, preferably at least about 91%, 92%, 93%, 94%, more preferably at least about 95%, or at least about 96%, 97%, 98%, 99% or 100% of the length of the shorter one of the two 16S sequences, and the window of comparison corresponds to the whole alignment length.

Preferably, the region of alignment, for example the region of alignment provided by a local alignment algorithm such as BLAST, will comprise at least 90% of the length of SEQ ID NO: 1, more preferably at least about 91%, 92%, 93%, 94%, even more preferably at least about 95%, or at least about 96%, 97%, 98%, 99% or 100% of the length of SEQ ID NO: 1. Preferably, the region of alignment, for example the region of alignment provided by a local alignment algorithm such as BLAST, will comprise at least 1390 contiguous (the term contiguous in this context does not exclude the presence of internal gaps in the alignment) nucleotides of SEQ ID NO: 1, more preferably at least 1200 contiguous nucleotides, or at least 1300 contiguous nucleotides, or at least 1400 contiguous nucleotides, such as at least 1410, at least 1420, at least 1430, at least 1440, at least 1450, or at least 1460 contiguous nucleotides of SEQ ID NO: 1. Particularly preferably, the region of alignment will comprises at least 1467, or in increasing order of preference, at least 1470, at least 1480, at least 1490, at least 1500, at least 1510, or at least 1520, or at least 1530, or at least 1540, or all 1544 contiguous nucleotides of SEQ ID NO: 1.

In certain examples, the bacterial strain can comprise a 16S polynucleotide having at least 99.80% sequence identity to SEQ ID NO: 1, or at least 99.85% sequence identity to SEQ ID NO: 1, or least 99.90% sequence identity to SEQ ID NO: 1, or at least 99.95% sequence identity to SEQ ID NO: 1., or the bacterial strain can comprise a 16S polynucleotide having 100.00% sequence identity to SEQ ID NO: 1.

In other examples, the bacterial strain can comprise a 16S polynucleotide which, when aligned over a region of alignment comprising at least 1467 contiguous nucleotides of SEQ ID NO: 1, or in increasing order of preference, over a region of alignment comprising at least 1470, at least 1480, at least 1490, at least 1500, at least 1510, at least 1520, at least 1530, at least 1540, or all 1544 contiguous nucleotides of SEQ ID NO: 1, will display no more than 3, preferably no more than 2, yet more preferably no more than 1, and most preferably no nucleotide mismatches and internal gaps with SEQ ID NO: 1. The mismatch or internal gap in this context refers to a single nucleotide mismatch or a gap that involves or spans a single nucleotide.

In other examples, the bacterial strain can comprise a 16S polynucleotide as set forth in (i.e., identical to) SEQ ID NO: 1, i.e., a 16S polynucleotide identical over the full length thereof to SEQ ID NO: 1.

In certain examples, the bacterial strain is a *Stenotrophomonas rhizophila* strain.

Most particularly, the bacterial strain as envisaged herein is the strain as deposited under the Budapest Treaty at the BCCM^{™} / LMG Bacteria collection (BCCM^{™}/LMG) on 24 December 2021 under Accession No. LMG P-32513 (with the following identification reference given to the deposited material by the depositor: **BML-B-A11E4(2)**)**.** Also described is functional mutant of BML-B-A11E4(2).

The term "functional mutant" means a bacterial strain directly or indirectly obtained by genetic modification (such as by random mutagenesis or by targeted genetic modification) of the respective referenced strain and retaining at least some extent of the activity of the referenced strain on the plant growth feature of interest, such as ability to or activity in increasing the biomass, height, and/or yield of the plant, preferably retaining at least 10%, such as at least 20%, at least 30%, or at least 40%, preferably at least 50%, such as at least 60%, at least 70%, or at least 80%, more preferably at least 90%, such as 100%, or even greater than 100% of the activity of the referenced strain on the plant growth feature of interest. The genetic modification of a functional mutant can be achieved through any means, such as, but not limited to, chemical mutagens, ionizing radiation, transposon-based mutagenesis, or via conjugation, transduction, or transformation using the referenced strains as either the recipient or donor of genetic material. In certain examples, the 16S rRNA gene sequence of the functional mutant remains identical to the 16S rRNA gene sequence of the referenced strain. Hence, the functional mutant may preferably comprise the 16S rRNA gene sequence as shown in SEQ ID NO: 1. In certain examples, the functional mutant comprises at most 10, such as in increasing order of preference, at most 9, 8, 7, 6, 5, 4, 3, 2, or at most 1 chromosomal loci (such as genes) whose nucleic acid sequence differs from (has been modified compared to) the sequence of the corresponding loci in the referenced strain, and/or the functional mutant comprises at most 10, such as in increasing order of preference, at most 9, 8, 7, 6, 5, 4, 3, 2, or at most 1 transgenic elements (such as transgenes) introduced into it and not present in the referenced strain. In certain examples, at most 10, such as in increasing order of preference, at most 9, 8, 7, 6, 5, 4, 3, 2, or at most 1 gene of the functional mutant carries a non-synonymous mutation not present in the reference strain.

Also disclosed is that bacteria of two or more bacterial strains as described in the present specification may be administered to the plant, the part thereof, the seed for growing the plant, or the locus of the plant.

The bacteria as described in the present disclosure may be administered to the plant, the part thereof, the seed for growing the plant, or the locus of the plant in conjunction with one or more additional plant-beneficial microorganism. As used throughout the present disclosure, the term "microorganism" or "microbe" refers to any strain, any species or taxon of microorganism, including, but not limited to, archaea, bacteria, microalgae, fungi (including mold and yeast species), mycoplasmas, microspores, nanobacteria, oomycetes, and protozoa. In some examples, a microbe or microorganism is a bacterial strain. In some examples, a microbe or microorganism is a fungal strain. In some examples, a microbe or microorganism is an endophyte, for example a bacterial or fungal endophyte, which is capable of living within a plant. In some examples, a microbe or microorganism encompasses individual cells (e.g., unicellular microorganisms) or more than one cell (e.g., multi-cellular microorganism).

Diverse plant-associated microorganisms can positively impact plant health and physiology in a variety of ways. In particular, plant-beneficial microorganisms, when administered to a plant, plant part, a seed for growing a plant, or a locus of a plant may improve one or more traits of agronomic importance in a plant, such as one or more plant growth features. Where the improved trait can be quantified, any extent of an improvement is contemplated. For example, a plant-beneficial microorganism may provide an improved trait of agronomic importance in a plant that is of at least 3%, between 3% and 5%, at least 5%, between 5% and 10%, least 10%, between 10% and 15%, for example at least 15%, between 15% and 20%, at least 20%, between 20% and 30%, at least 30%, between 30% and 40%, at least 40%, between 40% and 50%, at least 50%, between 50% and 60%, at least 60%, between 60% and 75%, at least 75%, between 75% and 100%, at least 100%, between 100% and 150%, at least 150%, between 150% and 200%, at least 200%, between 200% and 300%, at least 300% or more, when compared with a reference plant grown under the same conditions. By means of an illustration, a plant-beneficial microorganism may be capable of increasing nutrient uptake and/or nutrient use efficiency of a treated plant as compared to an untreated plant, increasing the nitrogen fixating capacities or phosphorus uptake of a treated plant as compared to an untreated plant, increasing the amount of biomass of a treated plant as compared to an untreated plant, increasing the number of tillers per plant of a treated plant as compared to an untreated plant, increasing growth and/or yield of a treated plant as compared to an untreated plant, and/or helping a treated plant overcome stress conditions, such as nutrient stress, compared to an untreated plant; and the like. Further traits of agronomic importance that can be improved by plant-beneficial microorganisms may include disease resistance, drought tolerance, heat tolerance, cold tolerance, salinity tolerance, metal tolerance, herbicide tolerance, chemical tolerance, improved water use efficiency, improved phosphorus solubilization, improved phosphorus mobilization, improved nitrogen utilization, improved nitrogen fixation, pest resistance, herbivore resistance, pathogen resistance, increase in yield, increase in yield under water-limited conditions, health enhancement, vigor improvement, growth improvement, improved plant emergence, photosynthetic capability improvement, nutrition enhancement, altered protein content, altered oil content, increase in biomass, increase in number of tillers per plant, increase in shoot length, increase in root length, improved root architecture, increase in seed weight, altered seed carbohydrate composition, altered seed oil composition, increase in radical length, delayed senescence, stay-green, altered seed protein composition, increase in dry weight of mature plant reproductive elements, increase in fresh weight of mature plant reproductive elements, increase in number of mature plant reproductive elements per plant, increase in chlorophyll content, reduced number of wilted leaves per plant, reduced number of severely wilted leaves per plant, increase in number of non-wilted leaves per plant, and/or improved plant visual appearance, and the like.

By means of an illustration and without limitation, plant-beneficial microorganisms may include mycorrhizal fungi, including endomycorrhizal fungi and ectomycorrhizal fungi, such as fungi belonging to the divisions Basidiomycota, Ascomycota, and Zygomycota, bacteria of the family *Rhizobiaceae,* bacteria of the genera *Frankic, Azotobacter, Azospirillum, Acetobacter, Azoarcus, Burkholderia, Herbaspirillum, Pseudomonas* (e.g., *Pseudomonas fluorescens, P. putida, P. gladioli*), *Bacillus* (*Bacillus subtilis, B. cereus, B. circulans*), further bacteria such as *Serratia marcescens, Flavobacterium* spp., *Alcaligenes* sp., *Agrobacterium radiobacter,* and others. In certain examples, the plant-beneficial microorganisms are selected from those disclosed in WO2018060519, WO2020161351, and WO2020161352.

The one or more additional plant-beneficial microorganism may be selected to improve the efficacy of the bacterial strain as taught herein, in particular efficacy in improving the plant growth feature acted on by the bacterial strain. Hence, also disclosed is a method of improving the efficacy of the bacterial strain as taught herein, comprising the selection of an additional plant-beneficial microorganism, whereby co-administration of the additional plant-beneficial microorganism with the bacterial strain or strains to a plant, plant part, or seed improves the plant growth feature. The administration of the plant-beneficial microorganism alone may but need not lead to an improvement in a plant trait.

The bacteria can be comprised in or be part of an agricultural active composition. Hence, the methods may entail administering or applying such an agricultural active composition to the plant, the part thereof (e.g., roots), the seed for growing the plant, or the locus of the plant (e.g., to soil or plant growth medium surrounding the plant).

The term "composition" generally refers to a thing composed of two or more components, and more specifically denotes a combination or mixture of two or more materials, such as elements, molecules, substances, and/or microorganisms, as well as reaction products and decomposition products formed from the materials of the composition. The term may be interchangeably used with the terms "formulation" or "preparation".

Agricultural active compositions typically comprise one or more agriculturally active ingredients and one or more agriculturally acceptable carrier or auxiliary. The terms "active ingredient" or "active component" can be used interchangeably and broadly refer to a material, such as an element, molecule, substance, and/or microorganism, which, when provided in an effective amount, achieves a desired outcome, such as achieves one or more effects on one or more traits of agronomic importance in plants. Typically, an active ingredient as intended herein may achieve such outcome(s) through interacting with and/or modulating the plant, part thereof, a seed for growing the plant, or the locus of the plant. The terms "agriculturally acceptable" or "agriculturally compatible" are consistent with the art and mean not deleterious to the recipient plant, such as not producing, having or causing any adverse effects when applied to a plant or to an organ, part or element of the plant, or adverse effects to the plant grown from that plant organ, part or element. The agriculturally active formulations may comprise materials which facilitate or enhance the stability, viability, storage, and/or administration of the bacterial strain(s) and/or plant-beneficial microorganism(s) as disclosed herein, and/or the colonization of the plant thereby.

Compositions as typically used herein may be liquid, semi-solid, or solid, and may include solutions or dispersions. Non-limiting examples of the compositions as taught herein may be soluble powders, soluble granules, wettable granules, tablet formulations, dry flowables, aqueous flowables, wettable dispersible granules, oil dispersions, suspension concentrates, dispersible concentrates, emulsifiable concentrates, aqueous suspensions, fertilizer granules, sprayables, and the like. A composition may be composed of components that are provided to an end user as a mixture, i.e., the composition components are already admixed. A composition may also be composed of components one or more of which are provided to an end user in a physically separated form (e.g., in separate containers or vials) from one or more other components of the composition, although typically as part of the same product package or dispensing device. By means of an example and without limitation, the composition may comprise one or more components provided in one container, and one or more components provided in another container. Such arrangement allows the end user to admix the components of the composition shortly before use. For example, the composition may comprise the bacteria and optionally further plant beneficial microorganism(s) as taught herein provided in one container, and one or more auxiliaries provided in another container, to be admixed by the end user before use.

In certain examples, the composition comprises one or more agriculturally acceptable auxiliary. The terms "auxiliary", "auxiliary agent", "additive", or "adjuvant" may be used interchangeably herein. The auxiliaries may be natural or synthetic organic or inorganic materials which facilitate the administration of actives to plants, plant parts, seeds, or plant growth loci. The auxiliaries may be one or more of as a solvent, a carrier, a surfactant, a sticker, an antifreeze agent, a thickener, a buffering agent, an antifoaming agent, an antioxidant, a preservative, an aroma, or a colorant. Suitable auxiliary agents and inert agents are known in the art and are commercially available. In general, the bacteria and optionally further plant-beneficial microorganism(s) can be combined with any solid, semi-solid or liquid additive customarily used for formulation purposes. A carrier is to be understood as meaning a natural or synthetic, organic or inorganic substance which is mixed or combined with the bacteria and optionally further plant-beneficial microorganism(s) for better applicability, in particular for application to plants or plant parts such as seeds. The carrier, which may be solid, semi-solid, or liquid, is generally inert and suitable for use in agriculture or horticulture. For instance, liquid carriers may include water, organic solvents, and mineral oils and vegetable oils. Suitable liquefied gaseous extenders or carriers are liquids which are gaseous at ambient temperature and under atmospheric pressure, for example aerosol propellants, such as butane, propane, nitrogen and carbon dioxide. A sticker is to be understood as meaning an additive or adjuvant to improve adhesive properties of the composition to the plant or part thereof. Suitable surfactants are emulsifiers, dispersants or wetting agents having ionic or nonionic properties, or mixtures of these surfactants. It is possible to use colorants such as inorganic pigments, for example iron oxide, titanium oxide, Prussian blue, and organic dyes, such as alizarin dyes, azo dyes and metal phthalocyanine dyes, and trace nutrients, such as salts of iron, manganese, boron, copper, cobalt, molybdenum and zinc. Stabilizers, such as low-temperature stabilizers, preservatives, antioxidants, light stabilizers or other agents which improve chemical and/or physical stability may also be present.

The bacteria and optionally further plant-beneficial microorganism(s) may be administered in combination with one or more other non-active or active ingredients that are non-toxic thereto. Such other ingredients may be an oil, an emulsifier, a spreader, a cryoprotectant, a binder, a dispersant, a surfactant, a buffer, a tackifier, a stabilizer, a microbial stabilizer, a bactericide (e.g., effective against bacteria other than those administered), a fungicide, a complexing agent, a herbicide, a nematicide, an insecticide, a molluscicide, an algicide, a fertilizer, a micronutrient fertilizer material, a plant growth regulator, a rodenticide, a preservative, a polymer, a desiccant, a nutrient, an excipient, a wetting agent, a salt, or any combination thereof.

The bacteria and optionally further plant-beneficial microorganism(s) may be co-administered and/or co-formulated with further biologicals or agrochemicals that stimulate plant growth and/or yield. Particular strains may be selected on the basis of their compatibility with commonly used biologicals or agrochemicals. Plants, particularly agricultural plants, can be treated with a vast array of biologicals or agrochemicals. In some cases, particular strain may be selected to be compatible with biologicals or agrochemicals with complexing properties, to facilitate persistence of the strain in the plant. There also exist many complexing agents that do not penetrate the plant, at least at a concentration sufficient to interfere with the administered bacteria. Where a systemic complexing agent is used in the plant, compatibility of the strain to be inoculated with such agents may be an important variable to consider. Purified bacterial strains that are compatible with biologicals or agrochemicals can be used to inoculate plants, plant elements or growth media according to the methods described herein.

Bactericide-compatible strains can also be isolated by selection on liquid medium. The culture of strains can be plated on petri dishes without any forms of mutagenesis; alternatively, strains can be mutagenized using any means known in the art. For example, strain cultures can be exposed to UV light, gamma-irradiation, or chemical mutagens such as ethylmethanesulfonate (EMS), ethidium bromide (EtBr), dichlorvos (DDVP), methyl methane sulphonale (MMS), triethylphosphate (TEP), trimethylphosphate (TMP), nitrous acid, or DNA base analogs, prior to selection on bactericide comprising media. Alternatively or in addition, where the mechanism of action of a particular bactericide is known, the target gene can be specifically mutated (either by gene deletion, gene replacement, site-directed mutagenesis, etc.) to generate a strain that is resilient against that particular chemical. The above-described methods can be used to isolate strains that are compatible with both bacteriostatic and bactericidal compounds. The biological or agrochemical compatible strains generated can be detected in samples. For example, where a transgene was introduced to render the strain compatible with the biological(s) or agrochemical(s), the transgene can be used as a target gene for amplification and detection by PCR. In addition, where point mutations or deletions to a portion of a specific gene or a number of genes results in compatibility with the biological(s) or agrochemical(s), the unique point mutations can likewise be detected by PCR or other means known in the art. Such methods allow the detection of the strain even if it is no longer viable.

In certain examples, the composition is a liquid composition. The compositions may be a ready-to-use composition which can be administered or applied with a suitable apparatus, or the composition may be a concentrate or a concentrated formulation which is to be diluted in a solvent, such as water or an aqueous solution or buffer prior to use. In certain further examples, the composition is an aqueous composition or a sprayable liquid or a concentrate or a spray, or a sprayable liquid or a dip.

The compositions as described herein can encompass an effective amount of the bacteria and optionally further plant-beneficial microorganism(s), i.e., an amount sufficient to elicit the desired outcome, such as one or more effects on one or more traits of agronomic importance in plants, such as an increase in the biomass of a plant, or yield of a plant, or both biomass and yield of a plant compared to an untreated plant, that is being sought by the user, in either a single or multiple doses, preferably in a single dose.

The composition can comprise the bacteria at a concentration of at least about 10 CFU/ml or at least about 10² CFU/ml. As used herein, a "colony forming unit" or "CFU" refers to a measure of viable microorganisms in a sample. A CFU is an individual viable cell capable of forming on a solid medium a visible colony whose individual cells are derived by cell division from one parental cell. The phrases "CFU", "CFU/ml", and "CFU/g" also encompass the reference to "spores", "spores/ml" or "spores/g", respectively, in case the microorganism lends itself to being administered in the form of spores.

In certain examples, the liquid composition comprises the bacteria at a concentration of at least about 10² CFU/ml, or of at least about 10³ CFU/ml, at least about 10⁴ CFU/ml, at least about 10⁵ CFU/ml, at least about 10⁶ CFU/ml, at least about 10⁷ CFU/ml, at least about 10⁸ CFU/ml, at least about 10⁹ CFU/ml, at least about 10¹⁰ CFU/ml, at least about 10¹¹ CFU/ml, or at least about 10¹² CFU/ml.

Also disclosed is that the liquid composition can comprise the bacteria at a concentration of from 1 x 10² CFU/ml to 1 x 10¹² CFU/ml, or from 1 x 10³ CFU/ml to 1 x 10¹¹ CFU/ml, or from 1 x 10³ CFU/ml to 1 x 10¹⁰ CFU/ml, or from 1 x 10⁴ CFU/ml to 1 x 10¹⁰ CFU/ml, or from 1 x 10⁵ CFU/ml to 1 x 10¹⁰ CFU/ml, or from 1 x 10⁶ CFU/ml to 1 x 10¹⁰ CFU/ml, or from 1 x 10⁶ CFU/ml to 1 x 10⁹ CFU/ml, or from 1 x 10⁷ CFU/ml to 1 x 10¹⁰ CFU/ml, or from 1 x 10⁷ CFU/ml to 1 x 10⁹ CFU/ml, or from 1 x 10⁸ CFU/ml to 1 x 10¹⁰ CFU/ml, or from 1 x 10⁸ CFU/ml to 1 x 10⁹ CFU/ml.

In certain examples, the composition is a non-liquid composition or a solid composition or a powdered composition or a powder. The term "powder" refers to a dry, bulk solid composed of many very fine particles that may flow freely when shaken or tilted.

In certain examples, the non-liquid composition, such as the powder, comprises the bacteria at an amount of at least about 10 CFU/g or of at least about 10² CFU/g. In certain examples, the non-liquid composition comprises the bacteria at an amount of at least about 10² CFU/g, or of at least about 10³ CFU/g, at least about 10⁴ CFU/g, at least about 10⁵ CFU/g, at least about 10⁶ CFU/g, at least about 10⁷ CFU/g, at least about 10⁸ CFU/g, at least about 10⁹ CFU/g, at least about 10¹⁰ CFU/g, at least about 10¹¹ CFU/g, or at least about 10¹² CFU/g.

In certain examples, the non-liquid composition comprises the bacteria at an amount of from 1 x 10² CFU/g to 1 x 10¹² CFU/g, or from 1 x 10³ CFU/g to 1 x 10¹¹ CFU/g, or from 1 x 10³ CFU/g to 1 x 10¹⁰ CFU/g, or from 1 x 10⁴ CFU/g to 1 x 10¹⁰ CFU/g, or from 1 x 10⁵ CFU/g to 1 x 10¹⁰ CFU/g, or from 1 x 10⁶ CFU/g to 1 x 10¹⁰ CFU/g, or from 1 x 10⁶ CFU/g to 1 x 10⁹ CFU/g, or from 1 x 10⁷ CFU/g to 1 x 10¹⁰ CFU/g, or from 1 x 10⁷ CFU/g to 1 x 10⁹ CFU/g, or from 1 x 10⁸ CFU/g to 1 x 10¹⁰ CFU/g, or from 1 x 10⁸ CFU/g to 1 x 10⁹ CFU/g.

Also disclosed is that the composition may comprise bacteria of two or more bacterial strains as described herein. In certain examples, the composition may comprise at least about 10² CFU/ml or at least about 10² CFU/g - such as the aforementioned more specific CFU/ml or CFU/g amount ranges - of bacteria of all the strains collectively or preferably of each of the strains individually and independently.

In certain examples, the composition comprises the one or more optional further plant-beneficial microorganism, such as cells or spores of one or more plant-beneficial microorganism strain, at a concentration or an amount, collectively or each individually and independently, of at least about 10² CFU/ml or 10² CFU/g, at least about 10³ CFU/ml or CFU/g, at least about 10⁴ CFU/ml or CFU/g, at least about 10⁵ CFU/ml or CFU/g, at least about 10⁶ CFU/ml or CFU/g, at least about 10⁷ CFU/ml or CFU/g, at least about 10⁸ CFU/ml or CFU/g, at least about 10⁹ CFU/ml or CFU/g, or at least about 10¹⁰ CFU/ml or CFU/g. More preferably, the composition comprises the one or more optional further plant-beneficial microorganism, such as cells or spores of one or more plant-beneficial microorganism strain, at a concentration or an amount, collectively or each individually and independently, of between 10³ to 10¹⁰ CFU/ml or CFU/g, between 10⁴ to 10¹⁰ CFU/ml or CFU/g, between 10⁵ to 10¹⁰ CFU/ml or CFU/g, between 10⁶ to 10¹⁰ CFU/ml or CFU/g, between 10⁶ to 10⁹ CFU/ml or CFU/g, between 10⁷ to 10⁹ CFU/ml or CFU/g, or between 10⁸ to 10⁹ CFU/ml or CFU/g.

Also disclosed is that the bacteria may be comprised by and administered as part of a bacterial population. A bacterial population may comprise bacteria of one or more bacterial strains as described herein, such as of two, three or more strains as described herein.

More generally, a bacterial population may comprise one or more, preferably two or more (e.g., 3, 4, 5, 6, 7, 8, 9, 10, 15, 20, 25 or more than 25) purified bacterial strains, wherein the strains may originate from different families of bacteria, or different genera of bacteria, or from the same genera but different species of bacteria. The taxonomically different bacterial strains can be obtained from the same cultivar of plant, different cultivars of the same plant, or different species of the same type of plant. The bacterial strains can be obtained from the soil wherein the plant is grown. In the case in which one or more, preferably two or more purified bacterial strains are used, each of the bacterial strains can have different properties or activities, e.g., produce different metabolites, produce different enzyme, confer different beneficial traits.

Also disclosed is that the bacterial population or composition may comprise bacteria of the one or more bacterial strain as described herein, and optionally one or more additional bacterial strain. In certain examples, the bacteria of the one or more bacterial strain as described herein may collectively constitute at least about 1% by CFU of all viable bacteria constituting the bacterial population or composition, such as at least about 2%, at least about 5%, at least about 10%, preferably at least about 20%, such as at least about 30%, or at least about 40%, more preferably at least about 50%, such as at least about 60%, at least about 70%, at least about 80%, at least about 90%, at least about 95%, at least about 99%, or even 100% by CFU of all viable bacteria constituting the bacterial population or composition.

Where the bacterial population or composition comprises bacteria of two or more bacterial strains as described herein, they may in certain examples be included in the population or composition in unequal amounts or preferably in about equal amounts. By means of an example and without limitation, the bacteria of each of the two or more bacterial strains as described herein may, each independently, constitute at least about 1% by CFU of all viable bacteria constituting the bacterial population or composition, such as at least about 2%, at least about 5%, at least about 10%, preferably at least about 20%, such as at least about 30%, or at least about 40%, more preferably at least about 50%, such as at least about 60%, at least about 70%, at least about 80%, at least about 90%, at least about 95%, or at least about 99% of all viable bacteria constituting the bacterial population or composition (where the sum of these amounts would exceed 100%, it shall be understood that the sum is capped at 100%).

Where the bacterial population or composition comprises bacteria of the one or more bacterial strain as described herein and bacteria of one or more additional bacterial strain, each bacterial strain may in certain examples be included in the population or composition in unequal amounts or preferably in about equal amounts. By means of an example and without limitation, the bacteria of each of the bacterial strains may, each independently, constitute at least about 1% by CFU of all viable bacteria constituting the bacterial population or composition, such as at least about 2%, at least about 5%, at least about 10%, preferably at least about 20%, such as at least about 30%, or at least about 40%, more preferably at least about 50%, such as at least about 60%, at least about 70%, at least about 80%, at least about 90%, at least about 95%, or at least about 99% of all viable bacteria constituting the bacterial population or composition (where the sum of these amounts would exceed 100%, it shall be understood that the sum is capped at 100%).

Also disclosed is that the concentration or amount of each isolated bacterial strain in the bacterial population or composition may be at least about 10² CFU/ml or CFU/g, at least about 10³ CFU/ml or CFU/g, at least about 10⁴ CFU/ml or CFU/g, at least about 10⁵ CFU/ml or CFU/g, at least about 10⁶ CFU/ml or CFU/g, at least about 10⁷ CFU/ml or CFU/g, at least about 10⁸ CFU/ml or CFU/g, at least about 10⁹ CFU/ml or CFU/g, or at least about 10¹⁰ CFU/ml or CFU/g. The concentration or amount of each isolated bacterial strain in the bacterial population or composition may also be between 10³ to 10¹⁰ CFU/ml or CFU/g, between 10⁴ to 10¹⁰ CFU/ml or CFU/g, between 10⁵ to 10¹⁰ CFU/ml or CFU/g, between 10⁶ to 10¹⁰ CFU/ml or CFU/g, between 10⁶ to 10⁹ CFU/ml or CFU/g, between 10⁷ to 10⁹ CFU/ml or CFU/g, or between 10⁸ to 10⁹ CFU/ml or CFU/g.

Also disclosed is a bacterial population, such as in accordance with the aforementioned explanations, comprising the bacterial strain as deposited under the Budapest Treaty at the BCCM^{™} / Laboratory of Microbiology (LMG) Bacteria collection (BCCM^{™}/LMG), on 24 December 2021 under Accession No. LMG P-32513 (with the following identification reference given to the deposited material by the depositor: **BML-B-A11E4(2)**).

Also disclosed is an agricultural active composition, such as in accordance with the aforementioned explanations, comprising the bacterial strain as deposited under the Budapest Treaty at the BCCM^{™} / Laboratory of Microbiology (LMG) Bacteria collection (BCCM^{™}/LMG), on 24 December 2021 under Accession No. LMG P-32513 (with the following identification reference given to the deposited material by the depositor: **BML-B-A11E4(2)**), or a functional mutant thereof.

Bacterial strains, combinations, populations, and compositions as discussed throughout the present disclosure can be employed in plants cultivation, in particular to facilitate an improvement or enhancement in the biomass, height, and/or yield of plants.

The terms "plant" or "plant element" as used herein encompasses whole plants, ancestors and progeny of the plants and plant parts, including seeds, shoots, stems, leaves, roots (including tubers), flowers, and tissues and organs. The terms "plant" or "plant element" also refer to plant cells, suspension cultures, callus tissue, embryos, meristematic regions, gametophytes, sporophytes, pollen and microspores. Hence, when the term "plant" or "plant element" is used herein, the term is intended to encompass "a plant, part thereof, or plant cell". The term "plant cell" may encompass a non-propagating plant cell.

The phrases "part of a plant" or "plant part" as used herein refer to any one or more portions of a plant, such as to any one or more of the seeds, shoots, stems, leaves, roots (including tubers), flowers, and tissues and organs of a plant, such as for example meristematic tissue, ground tissue, vascular tissue, dermal tissue, etc. In addition, a "plant part" is intended to generically reference any part of a plant that is able to initiate other plants via either sexual or asexual reproduction of that plant, for example but not limited to: seed, seedling, root, shoot, cutting, scion, graft, stolon, bulb, tuber, corm, keikis, or bud.

The part of a plant may be any one or more of the seeds, shoots, stems, leaves, roots (including tubers), or flowers. The part of a plant may be the seeds. In other examples, the part of a plant may be the shoots, stems, or leaves. In yet other examples, the part of a plant may be the roots (including tubers). The part of a plant may also be tissues or organs of a plant.

The seeds, shoots, stems, leaves, roots (including tubers), flowers, tissues or organs of the plant, when treated according to the methods as taught herein, may be attached to (e.g., growing on) the whole plant. In certain examples, the seeds, shoots, stems, leaves, roots (including tubers), flowers, tissues or organs of the plant, when treated according to the methods as taught herein, may be detached from (e.g., not growing on) the whole plant. For instance, seeds may be detached from (e.g., not growing on) the whole plant when treated according to the methods as taught herein.

In some examples, plants may include wild plants and domesticated varieties or may be developed by any technique, including but not limited to directed evolution, selection, marker assisted selection, hybridization, outcrossing, backcrossing, in-breeding, polyploidization, reverse breeding, doubled haploids, induced mutation, other genetic or epigenetic modifications, and combinations thereof.

The phrases "locus of a plant" or "locus of growth of a plant" as used herein refers to an area in close proximity of a plant (including parts thereof such as a seed). For instance, the locus of a plant may be a circular area around the plant, e.g., around the stem of a plant or around a seed, such as a circular area having a diameter of at most 1 meter, for instance at most 50 centimeters (cm), at most 40 cm, at most 30 cm, at most 20 cm, at most 10 cm, or at most 5 cm, around the plant, e.g., around the stem of a plant or around a seed. The locus of growth may include the growth medium (e.g., soil, hydroponic medium, or hydroculture medium) for cultivating the plant.

The reference to plants includes any plants. The plants may be an angiosperm. Particularly preferred are agricultural plants. The terms "agricultural plants", "crops" or "plants of agronomic importance" as used herein include plants that are cultivated by humans for but not limited to food, feed, fiber, fuel, gardening, and/or industrial purposes.

In certain examples, the plant is a monocotyledon. The terms "monocotyledon" or "monocot" refer to flowering plants (angiosperms) whose seeds typically contain only one embryonic leaf or cotyledon.

In certain preferred examples, the plant is a cereal plant. The terms "cereal" or "cereal plant" refer to any grass cultivated for the edible components of its grain (caryopsis), composed of the endosperm, germ, and bran.

In certain preferred examples, the plant is selected from the group consisting of wheat, maize, barley, rice, millet, rye, triticale, sorghum, emmer, spelt, einkorn, teff, milo, and oats. In certain particularly preferred examples, the plant is wheat or maize. In certain particularly preferred examples, the plant is wheat (*Triticum aestivum* and related varieties). In further particularly preferred examples, the plant is maize (*Zea mays* and related varieties).

In other examples, the plant is a dicotyledon. The terms "dicotyledon" or "dicot" refer to flowering plants (angiosperms) whose seeds typically contain two embryonic leaves or cotyledons.

The plant may be a non-modified plant or a modified plant. As used herein, a plant may be "modified" when it comprises an artificially introduced genetic or epigenetic "modification". In some examples, the modification is introduced by a genome engineering technology. In some examples, the modification is introduced by a targeted nuclease. In some examples, targeted nucleases include, but are not limited to, transcription activator-like effector nuclease (TALEN), zinc finger nuclease (ZNF), clustered regulatory interspaced short palindromic repeats (CRISPR), CRISPR/Cas9, CRISPR/CPFL and combinations thereof. In some examples, the modification is an epigenetic modification. In some examples, the modification is introduced by treatment with a DNA methyltransferase inhibitor such as 5-azacytidine, or a histone deacetylase inhibitor such as 2-amino-7-methoxy-3H-phenoxazin-3-one. In some examples, the modification is introduced via tissue culture. In some examples, a modified plant may comprise a transgene. In certain examples, the plant may be a non-transgenic plant or a transgenic plant.

In certain preferred examples, the plant may be a non-transgenic plant or a transgenic plant. The terms "recombinant", "transgenic" or "transgene" as used herein, for example with regard to a plant, refer to those plants brought about by recombinant methods in which a nucleic acid sequence and/or genetic control sequence(s) which are operably linked to the nucleic acid sequence are not located in their natural genetic environment. The natural genetic environment is understood as meaning the natural genomic or chromosomal locus in the original plant. A naturally occurring nucleic acid sequence (e.g., a naturally occurring combination of the native promoter of a nucleic acid sequence, the corresponding native nucleic acid sequence encoding a protein, and the native transcription termination sequence of a nucleic acid sequence) becomes a recombinant nucleic acid when this nucleic acid is not integrated in the natural genetic environment but in a different genetic environment as a result of an isolation of said nucleic acid from its natural genetic environment and re-insertion at a different genetic environment.

In certain examples, the plant may be free of disease and/or pathogen pressure and/or pest organisms. In other examples, the plant may be affected with disease and/or pathogen pressure and/or pest organisms.

The products, methods and uses as taught herein can provide for advantages in plants treated therewith relative to untreated plants. An "untreated plant" refers to a plant of the same species as (e.g., which is isogenic to or genetically identical to) and grown under substantially the same conditions as (e.g., for the same amount of time, in the same climate, and cultivated according to the same methods using the same materials, with biomass, yield and other characteristics being measured according to the same methods) a plant which has been administered the bacterial strain(s) (for reasons of brevity, the mention of bacterial strain(s) henceforth encompasses the one or more bacterial strain as envisaged herein, as well as the bacterial strain combinations and bacterial populations as disclosed herein, as well as the compositions comprising these, insofar the context does not indicate otherwise; these may also contain the optional further plant-beneficial microorganism(s)), except that the untreated plant has not been administered said bacterial strain(s) to the plant, a part thereof, a seed for growing the plant, or locus of the plant. The term may be used synonymously to "reference plant" or "reference", a plant genetically identical to and handled in substantially identical ways to a treated plant, with the exception of the treatment under investigation, and which thus offers a meaningful and informative control for detecting the effects of said treatment. A treated plant and a control reference plant can thus be exposed to substantially the same environmental conditions. By means of an example, the treated plant and reference plant can both be observed under substantially identical conditions of drought stress, or the treated plant and reference plant can both be observed under substantially identical conditions of no drought stress.

In one example, two genetically identical maize plant embryos may be separated into two different groups, one receiving a treatment (such as administration of the bacterial strain(s)) and one control, e.g., reference, that does not receive such treatment. Any phenotypic differences between the two groups may thus be attributed solely to the treatment and not to any inherency of the plant's genetic makeup. In another example, two genetically identical wheat seeds may be treated with a composition, one that introduces a bacterial population and one that does not. Any phenotypic differences between the plants derived from (e.g., grown from or obtained from) those seeds may be attributed to the bacterial treatment.

The term "untreated seed" refers to a seed of the same species as (e.g., which is isogenic to or genetically identical to) and obtained under substantially the same conditions (e.g., plants from which the seeds are obtained are grown for the same amount of time, in the same climate, and cultivated according to the same methods using the same materials, seeds are stored under the same conditions) as a seed which has been administered the bacterial strain(s), except that the untreated seed has not been administered said bacterial strain(s).

The term "plant growth feature" is intended to broadly encompass any feature that relates in some way to plant growth. The feature may relate to or be observable with respect to an individual plant or to a population of plants. Examples of such features include, without limitation, plant wet or dry biomass, plant height, plant size, emergence %, emergence date, canopy cover, flowering status, seed yield, grain yield, fruit yield, number of tillers per plant, shoot length, root length, root architecture, seed weight, senescence, stay-green, number of mature plant reproductive elements per plant, visual appearance, etc.

The reference to an improvement encompasses any qualitative or quantitative change or modification in a plant growth feature that is industrially beneficial, in particular in the context of agriculture. To the extent a plant growth feature is quantifiable, an improvement may be synonymous to an increase or a reduction in that quantity, depending on the nature of the plant growth feature. By means of an example and without limitation, an increase may be desired in quantifiable features such as plant wet or dry biomass, plant height, plant size, canopy cover, seed yield, grain yield, fruit yield, number of tillers per plant, shoot length, root length, seed weight, etc.

In certain examples, the plant growth feature comprises or is biomass, height, yield, or any combination thereof.

As used herein, the "biomass" of a plant refers to the amount (e.g., as determined by mass or weight, e.g., measured in grams of air-dry or wet tissue) or quantity (numbers) of tissue produced from the plant. Unless specified otherwise, biomass comprises both aboveground biomass (i.e., aerial biomass, including but not limited to stem, leaves, fruits, and/or seeds) and/or belowground biomass (i.e., roots). The biomass refers to the biomass at a given time. The biomass of a plant that has been administered the bacterial strain(s) can be measured according to known methods including weighing. Biomass may be given as weight per unit area. The term may also refer to all the plants or species in the community (community biomass). In certain examples, an increase in the biomass of a plant or part thereof may include an increase in the dry biomass, the wet biomass, the number of tillers, the height of the plant, the plant yield, the seed yield, the fruit yield, or a combination thereof.

The dry biomass of the plant may be measured according to the dry weight (DW) of the plant or part thereof in grams. The dry weight may be determined after drying the plant or part thereof until no residual water is left, e.g., after drying at 60°C for 1 week. The wet biomass of the plant may be measured according to the wet or fresh weight of the plant or part thereof in grams. The number of tillers may be determined by counting the tillers. The height of the plant may be determined by measuring the height.

As used herein the phrase "yield" or "plant yield" refers to the amount, mass or weight (e.g., as determined by weight or size) or quantity (numbers) of tissues or organs produced per plant, per growing area, and/or per growing season.

The plant yield may be affected by various parameters including, but not limited to, plant biomass; plant vigor; growth rate; seed yield; seed or grain quantity; seed or grain quality; oil yield; content of oil, starch and/or protein in harvested organs (e.g., seeds or vegetative parts of the plant); number of flowers (florets) per panicle (expressed as a ratio of number of filled seeds over number of primary panicles); harvest index; number of plants grown per area; number and size of harvested organs per plant and per area; number of plants per growing area (density); number of harvested organs in field; total leaf area; carbon assimilation and carbon partitioning (the distribution/allocation of carbon within the plant); resistance to shade; number of harvestable organs (e.g. seeds), seeds per pod, weight per seed; and modified architecture.

As used herein the phrases "seed yield" or "grain yield" refer to the number or weight of the seeds per plant, seeds per pod, or per growing area or to the weight of a single seed. Hence seed yield can be affected by seed dimensions (e.g., length, width, perimeter, area and/or volume), number of (filled) seeds and seed filling rate. An increased seed yield per growing area could be obtained by increasing seed yield per plant, and/or by increasing number of plants grown on the same growing area.

The term "seed" (also referred to as "grain" or "kernel") as used herein refers to a small embryonic plant enclosed in a covering called the seed coat (usually with some stored food). The seed is the product of the ripened ovule of gymnosperm and angiosperm plants which occurs after fertilization and some growth within the mother plant. The terms "seed", "plant seed", or "seed for growing the pant" may be used interchangeably herein.

The biomass of a plant and/or the yield of a plant that has been administered the bacterial strain(s) can be measured at a timepoint that is between about 7 days to about 350 days, about 7 days to about 300 days, about 7 days to about 250 days, about 7 days to about 200 days, about 7 days to about 150 days, or about 10 days to about 100 days, such as about 15 days to about 75 days, about 20 days to about 60 days, or about 25 days to about 50 days following administration of said bacterial strain(s) to the plant. The biomass, height, and/or yield of a plant, such as a cereal plant, that has been administered the bacterial strain(s) can be measured at the time that the plant is harvested to collect its grain or produce, i.e., at the time that the mature plant, such as a cereal plant, e.g., a wheat or maize plant, is gathered from a field. The biomass of a plant and/or the yield of a plant that has been administered the bacterial strain(s) vs. a reference plant not so treated would be measured at the same time point.

The plant growth feature may comprise dry biomass, wet biomass, plant height, seed or grain yield, or any combination thereof. In certain examples, the present disclosure allows to improve or increase any one or more and preferably all of these features.

The term "increase" as used herein is intended to be synonymous with terms such as "upregulate", "enhance", "stimulate", or "boost". An increase in the plant biomass, height, and/or yield can be in the whole plant or in any part thereof such as aboveground (harvestable) parts, vegetative biomass, roots, fruits, or seeds. Any extent or degree of such increase is contemplated herein, in particular any agronomically meaningful increase. Typically, the term may in appropriate contexts, such as in experimental or agricultural contexts, denote a statistically significant increase relative to a reference. The skilled person is able to select such a reference, as also discussed elsewhere in this specification. For example, such increase may fall outside of error margins for the reference (as expressed, for example, by standard deviation or standard error, or by a predetermined multiple thereof, e.g., ±1xSD or ±2xSD, or ±1xSE or ±2xSE). Accordingly, while the respective improvements or increases may be observable at the level of individual plants, they are more usefully evaluated by comparing relevant population characteristics, such as average or median values of the respective traits, obtained using sample sizes of treated vs. untreated plants that allow for statistically meaningful conclusions, such as for example shown in the Examples section.

In certain examples, the biomass, height, and/or yield of a plant may each independently be increased by at least about 1% relative to (i.e., compared with) (i.e., the biomass of a plant may be at least about 1.01-fold) the biomass, height, and/or yield of an untreated plant, such as preferably by at least about 2% (i.e., 1.02-fold), by at least about 3% (i.e., 1.03-fold), by at least about 5% (i.e., 1.05-fold), by at least about 10% (i.e., 1.10-fold), by at least about 15% (i.e., 1.15-fold), by at least about 20% (i.e., 1.20-fold), or more, such as by at least about 25% (i.e., 1.25-fold), by at least about 30% (i.e., 1.30-fold), by at least about 35% (i.e., 1.35-fold), by at least about 40% (i.e., 1.40-fold), by at least about 45% (i.e., 1.45-fold), by at least about 50% (i.e., 1.50-fold), or more. For example, the biomass, height, and/or yield of a plant may be increased by between 2% and 5%, between 5% and 10%, between 10% and 15%, between 15% and 20%, between 20% and 30%, between 30% and 40%, or between 40% and 50% relative to the biomass, height, and/or yield of an untreated plant. Such enhanced biomass, height, and/or yield may advantageously reduce or even abolish the need to treat the plants with agrochemicals such as fertilizers in the field and thereby advantageously leads to more sustainable agriculture. As said above, these percentages or fold increases may conveniently reflect the relationships between averages for the respective traits in the treated vs. untreated populations, as determined by evaluating representative population samples.

In certain examples, the seed or grain yield of the plant may be increased, such as increased by the aforementioned percentages or fold change.

As set forth elsewhere in this document, the bacterial strain(s) can thus be administered to the plant, a part of the plant, a seed for growing the plant, or locus of the plant in an amount effective to produce an improved plant growth feature, such as an increased biomass , height, and/or yield in the plant compared to an untreated plant.

The phrase "administering" generally refers to man- and/or machine-driven or effected disposing, applying, delivering, or providing of a recited object, such as the bacterial strain(s), to a recipient entity, such as the plant, a part thereof, a seed for growing the plant, or locus of the plant. The bacterial strain(s) as taught herein may be administered by any known method wherein all or part of the plant is treated, such as by root, seed, or foliar inoculation. For example, the administration can be to the aerial portions of a plant, such as the leaves and stem, to the roots of the plant, to the seed of the plant prior to planting the seed in soil, or to the soil or plant growth medium surrounding the plant or plant seed. Application methods such as spraying, coating, covering, contacting, and/or immersion can be adopted. In certain examples, application may be to a surface, such as to the surface of growth medium (such as soil), plant, plant part, seeds, harvested crop, harvested seed crop, stored crops or crop parts. In other examples, the administration may be to the plant, part thereof, or locus of the plant, present on the field. The terms "field" or "agricultural field" may be used interchangeably herein and refers to an area of land used for agricultural purposes such as cultivating crops, such as cultivating wheat plants or maize plants.

In certain examples, the bacterial strain(s) may be applied to the part of the plant, when forming part of the plant. For instance, they may be applied to the part of the plant, such as to the leaf, when the part of the plant, such as the leaf, is present on or attached to (e.g., is growing on) the plant.

In certain examples, the bacterial strain(s) may be applied to any one or more of the seeds, shoots, stems, leaves, roots (including tubers), flowers, tissues, or organs of a plant. In certain examples, the bacterial strain(s) may be applied to (e.g., sprayed on) the whole of the above-ground part of the plant. Accordingly, in certain examples, the bacterial strain(s) may be applied to (e.g., sprayed on) any one or more of the shoots, stems, leaves, or flowers of the plant. Preferably, the bacterial strain(s) may be applied to (e.g., sprayed on) any one or more of the shoots, leaves, or flowers of the plant. In certain examples, the bacterial strain(s) may be applied to (e.g., sprayed on) the shoots of the plant.

In other examples, the bacterial strain(s) may be administered to the locus of the plant, such as by inoculating the growth medium. Hence, in certain examples, the method comprises inoculating soil or a plant growth medium with the bacteria and growing the plant in said soil or medium.

The terms "growth medium" or "plant growth medium" as used herein refer to a substrate or medium for culturing plants. The growth medium may be soil, compost, peat, coco-coir, wood fibers, a soil-mimicking substrate such as mineral lava or basalt substrate, textile, or a soil-less substrate. The grown medium may also be sand, gravel, polysaccharide, mulch, peat moss, straw, logs, clay, or a combination thereof. The plant growth medium may also include a hydroculture system or an *in vitro* culture system. Hence, in certain examples, the plant growth medium is a hydroponic medium or a hydroculture medium. The skilled person understands that different types of growth media may be used for growing different types of plants. Inoculating a plant growth medium can be performed, by way of example using a liquid, a powder, a granule, a pellet.

Hydroculture, also encompassing hydroponics, is the growing of plants in a soil-less medium or an aquatic based environment, while *in vitro* culture system refers to the growing of plants or explants on or in a recipient with synthetic medium, in sterile conditions, in a controlled environment and in reduced space. Explants refer to parts of a plant, from all the aerial part to isolated cells, as parts of leaves, of roots, seeds, bulbs, tubers, buds. The inoculation of the plant growth medium with the bacterial strain(s) may be performed before, during and/or after sowing or before, during and/or after the start of the plant growth cycle in case of hydroculture or *in vitro* culture. The inoculation can be performed once or multiple times during the plant growth cycle.

Sprayable liquids may be applied by spraying the plant, part thereof, or locus of the plant by conventional spraying equipment as known in the art, such as airplanes, backpack sprayers, tractor mounted boom sprayers etc.

Application of the bacterial strain(s) to the plant, part thereof, or locus of growth of the plant may be carried out directly or by action on their surroundings or habitat using customary treatment methods, for example by dipping, drenching, spraying, coating, atomizing, irrigating, evaporating, dusting, fogging, broadcasting, foaming, painting, spreading-on, watering (drenching) or drip irrigating. The method may comprise spraying, sprinkling, showering, spritzing, spreading in droplets, spattering; dispersing, diffusing, or douching the plant, part thereof, or locus of growth of the plant with the bacterial strain(s).

The bacteria of the one or more bacterial strain as taught herein may be applied to a locus where plants are or are to be grown, such as upon soil, such as upon a field or within a greenhouse, in an amount of from about 1 x 10⁸ CFU/hectare to about 1 x 10¹⁴ CFU/ha, such as from about 1 x 10⁹ CFU/hectare to about 1 x 10¹³ CFU/ha, or from about 1 x 10¹⁰ CFU/hectare to about 1 x 10¹² CFU/ha, preferably about 1 x 10¹¹ CFU/hectare

The application may be one-time (single) administration, repeated administration (i.e., more than one time administration) at the same or varying time intervals, or continuous administration. The bacterial strain(s) can be administered at any point in the life cycle of the plant (e.g., before or after germination). For example, administration can be to a plant's seed prior to planting the seed in soil and prior to germination. Alternatively, administration can be to the plant (e.g. a seedling), the seed of the plant, or the soil surrounding the plant after germination has occurred. Once treated with the bacterial strain(s), seeds can be planted in soil and cultivated using conventional methods for generating plant growth.

The bacterial strain(s) may be applied at a temperature (e.g., air temperature) in the range from - 1°C to 30°C. The application may be at a temperature in the range from 0°C to 30°C, from 1°C to 30°C, from 5°C to 25°C, or from 10°C to 20°C.

The bacterial strain(s) may be applied to the part of the plant, when not forming part of the plant. For instance, they may be applied to the part of the plant, such as to a seed for growing the plant, when the part of the plant, such as the seed, is not present on or is detached from (e.g., is not growing on) the plant. For instance, they may be applied to seeds after they have been harvested from (e.g. mechanically or manually separated from) the plant. In certain examples, the method may comprise administering the bacterial strain(s) to a seed of the plant, e.g., prior to planting the seed or with the seed at planting. Hence, in certain examples, the method comprises administering the bacterial strain(s) to a seed of the plant. In certain examples, the bacterial strain(s) are administered to the seed of the plant prior to planting the seed, or with the seed at planting, or after planting the seed and before germination of the seed.

In certain examples, the purified bacterial strain(s) are capable of colonizing plants. Successful colonization can be confirmed by detecting the presence of the strain within the plant. For example, after applying the strain to the plant parts, high titers of the strain can be detected in the roots and shoots of the plants that germinate from said plant parts such as seeds. Detecting the presence of the strain inside the plant can be accomplished by measuring the viability of the strain after surface sterilization of the plant element or the plant: strain colonization results in an internal localization of the strain, rendering it resistant to conditions of surface sterilization. The presence and quantity of strain can also be established using other means known in the art, for example, immunofluorescence microscopy using microbe-specific antibodies, or fluorescence in situ hybridization. Alternatively, specific nucleic acid probes recognizing conserved sequences from a strain can be employed to amplify a region, for example by quantitative PCR, and correlated to CFUs by means of a standard curve.

Hence, microorganisms such as bacteria are said to colonize a plant, plant part, root or seed, when they can exist in relationship with a plant or plant part during at least part of either the plant's or the microorganism's life cycle. In certain examples, microorganisms such as bacteria are said to colonize a plant when they can be stably detected within the plant or plant part over a period time, such as one or more days, weeks, months or years. The compositions and methods described herein may comprise one or a plurality of bacterial strains as taught herein and optionally one or more further plant-beneficial microorganism in amounts effective to colonize a plant.

The strains described herein may be capable of moving from one tissue type to another. For example, the detection and isolation of strains within the mature tissues of plants after treating the exterior of a plant part demonstrates their ability to move from the plant part into the vegetative tissues of a maturing plant. Therefore, in some examples, the population of bacterial strains is capable of moving from the plant element exterior into the vegetative tissues of a plant. In other examples, the strain that is disposed onto the plant element of a plant is capable, upon germination of the plant part into a vegetative state, of localizing to a different tissue of the plant. For example, strains can be capable of localizing to any one of the tissues in the plant, including: the root, adventitious root, seminal root, root hair, shoot, leaf, flower, ear, spike, spikelet, bud, tassel, meristem, pollen, pistil, ovaries, stamen, fruit, stolon, rhizome, nodule, tuber, trichome, guard cells, hydathode, petal, sepal, glume, rachis, vascular cambium, phloem, and xylem. In an example, the strain is capable of localizing to the root and/or the root hair of the plant. In another example, the strain is capable of localizing to the photosynthetic tissues, for example, leaves and shoots of the plant. In other cases, the strain is localized to the vascular tissues of the plant, for example, in the xylem and phloem. In still another example, the strain is capable of localizing to the reproductive tissues (flower, pollen, pistil, ovaries, stamen, fruit, spike, spikelet) of the plant. In another example, the strain is capable of localizing to the root, shoots, leaves and reproductive tissues of the plant. In still another example, the strain colonizes a fruit or plant element tissue of the plant. In still another example, the strain is able to colonize the plant such that it is present in the surface of the plant (i.e. its presence is detectably present on the plant exterior). In still other examples, the strain is capable of localizing to substantially all, or all, tissues of the plant. In some cases, strains are capable of replicating within the host plant and colonizing the plant.

In further examples, following administration of the bacterial strain(s), the plants are cultivated under conditions to promote plant growth and development. In other words, the present methods may further comprise cultivating the plant under conditions to promote plant growth and development.

In certain examples, the method comprises administering the bacterial strain(s) to a seed of the plant, a whole plant, or a seedling, and optionally cultivating the seed, whole plant, or seedling under conditions to promote plant growth and development. In certain examples, the method comprises administering the bacterial strain(s) to a seed of the plant, and optionally cultivating the seed under conditions to promote plant growth and development. Hence, in such latter examples, the plant is grown from a seed, in particular a seed planted in said soil or plant growth medium.

Cultivating the seed under conditions promoting plant growth and development, may but need not include growth to maturity and/or regeneration.

In certain examples, the method may comprise further propagating the plant treated with the bacterial strain(s). Accordingly, the disclosure describes a method for increasing biomass, height, and/or yield of a plant grown from a seed compared to a plant grown from an untreated seed, the method comprising administering the bacterial strain(s) to the seed for growing the plant. The disclosure also describes the use of the bacterial strain(s) for increasing biomass, height, and/or yield of a plant grown from a seed compared to a plant grown from an untreated seed.

Also described herein is a method of treating a seed of a plant comprising inoculating the seed with bacteria of one or more bacterial strain as taught herein, such that the bacteria colonize a plant germinated from the inoculated seed and/or the soil or plant growth medium surrounding the growing plant, whereby a plant growth feature of the plant, such as the biomass, height, and/or yield of the plant, is improved compared to a plant germinated from an untreated seed. In certain examples, the seed is coated with the bacteria, incubated with the bacteria, or planted near the bacteria. In other examples, the seed is further inoculated with one or more additional plant-beneficial microorganism as taught herein.

The bacteria of one or more bacterial strain as taught herein may be contacted with seeds in an amount of between about 1 x 10⁶ CFU/kg seeds to about 1 x 10¹² CFU/kg seed, such as between about 1 x 10⁷ CFU/kg seeds to about 1 x 10¹¹ CFU/kg seeds, or between about 1 x 10⁸ CFU/kg seeds to about 1 x 10¹⁰ CFU/kg seeds, or between about 1 x 10⁹ CFU/kg seeds to about 1 x 10¹⁰ CFU/kg seeds, preferably about 2.5 x 10⁹CFU/kg. In certain examples, seeds may be treated with cultivated bacteria of one or more bacterial strain as taught herein, such as with a bacterial sample or culture showing Optical Density (OD) between about 0.1 and about 1 at wavelength 600 nm, at an amount of between about 0.1 l to about 5 l of the culture per kg of seeds, such as about 4.6 l.

The bacteria of one or more bacterial strain as taught herein may be coated on or present on or inoculated into seeds at a quantity of, on average, at least 10 CFU per seed, preferably at least 100 CFU per seed, more preferably at least 500 CFU per seed, and even more preferably at least 1000 CFU per seed, such as between 1 x 10³ and 1 x 10⁵ CFU per seed, or between 1 x 10³ and 1 x 10⁴ CFU per seed, or for example at least 1 x 10⁴ CFU per seed, or 1 x 10⁵ CFU per seed or 1 x 10⁶ CFU per seed, such as between 1 x 10⁵ and 1 x 10⁷ CFU per seed, preferably about 1 x 10⁶ CFU per seed. Certain examples thus describe a plant seed comprising at least 10 CFU preferably at least 100 CFU, more preferably at least 500 CFU, and even more preferably at least 1000 CFU, such as more preferably at least 1 x 10⁴ CFU, or 1 x 10⁵ CFU, or 1 x 10⁶ CFU, such as between 1 x 10⁵ and 1 x 10⁷ CFU, preferably about 1 x 10⁶ CFU, of the bacteria as taught herein. For example, the seed may be coated with the bacterial cells or the composition as taught herein.

The bacteria of one or more bacterial strain as taught herein can be cultured on a culture medium or can be adapted to culture on the culture medium. Said culture medium is sterile prior to being inoculated with the bacterial strain and comprises all nutrients for growth and maintenance of the strain on the culture medium. In addition, the culture medium can be in a solid, semi-solid or liquid form.

The bacteria of one or more bacterial strain as taught herein may be administered as a bacterial sample, culture, or inoculum (e.g., showing Optical Density (OD) between about 0.1 and about 1 at wavelength 600 nm, for example measured using an Implen GmbH (Munich, Germany) spectrophotometer, spray dried bacteria, freeze dried bacteria, suspension of spray dried or freeze dried bacteria, etc.

The bacteria of one or more bacterial strain as taught herein may thus be administered as a whole cell broth, comprising the bacteria as well as the medium in which the bacteria have been grown.

Also described is a plant or part thereof treated with the bacterial strain(s). Also described is a plant or part thereof heterologously disposed with the bacteria as taught herein. The plant part may be a seed, such as a seed coated with the bacteria or the composition. The plant or part thereof or a plant grown from said plant part can display increased biomass as compared to an untreated plant or part thereof.

Hence, also disclosed is a plant seed, preferably a crop plant seed (e.g., the seed of a wheat plant or maize plant), treated with, such as coated with, the bacterial strain(s), e.g., such that all or part of the seed has a coating or film comprising the bacteria. The plant grown from the treated seed can display increased biomass as compared to a plant grown from an untreated seed.

Further disclosed is a plant grown from a plant or part thereof (e.g., seed) treated with the bacterial strain(s).

Bacteria of the one or more strain as taught herein may, collectively or preferably each of the strains individually and independently, be present in an amount of at least about 10² CFU per treated plant or part thereof. Bacteria of the one or more strain as taught herein may, collectively or preferably each of the strains individually and independently, be present in an amount of at least about 10² CFU per plant grown from the treated plant or part thereof such as from a treated seed.

Preferably, bacteria of the one or more strain as taught herein may, collectively or preferably each of the strains individually and independently, be present on the plant or part thereof in an amount effective to be detectable within a target tissue of the mature plant selected from a fruit, a seed, a leaf, or a root, or portion thereof. For example, in an amount of at least about 100 CFU, between 100 and 200 CFU, at least about 200 CFU, between 200 and 300 CFU, at least about 300 CFU, between 300 and 400 CFU, at least about 500 CFU, between 500 and 1,000 CFU, at least about 1,000 CFU, between 1,000 and 3,000 CFU, at least about 3,000 CFU, between 3,000 and 10,000 CFU, at least about 10,000 CFU, between 10,000 and 30,000 CFU, at least about 30,000 CFU, between 30,000 and 100,000 CFU, at least about 10⁵ CFU, between 10⁵ and 10⁶ CFU, at least about 10⁶ CFU or more in the mature plant.

The plant or part thereof, such as a seed, treated with (e.g. coated with) the bacteria as taught herein may be shelf-stable. The bacterial strain may be shelf-stable, where at least 0.01%, of the CFUs are viable after storage in desiccated form (i.e., moisture content of 30% or less) for 1, 2, 3, 4, 5, 6, 7, 8, 9, 10 or greater than 10 weeks at 4°C or at room temperature. Optionally, a shelf-stable composition comprising the bacteria may be in a dry composition, a powder composition, or a lyophilized composition. The composition may be formulated to provide stability for the strains. The plant or part thereof, such as a seed, treated with (e.g. coated with) the bacteria may be substantially stable at temperatures between about -20°C and about 50°C for at least about 1, 2, 3, 4, 5, or 6 days, or 1, 2, 3 or 4 weeks, or 1, 2, 3, 4, 5, 6, 7, 8, 9, 10, 11 or 12 months, or one or more years. The plant or part thereof, such as a seed, treated with (e.g. coated with) the bacteria may be substantially stable at temperatures between about 4°C and about 37°C for at least about 5, 10, 15, 20, 25, 30 or greater than 30 days. Preferably the plant or part thereof, such as a seed, treated with (e.g. coated with) the bacteria is substantially stable at temperatures between about 4°C and about 37°C for at least one year or greater than one year.

In certain examples, the plant or part thereof, such as a seed, treated with (e.g. coated with) the bacteria are confined within a suitable container, such as an object selected from the group consisting of: bottle, jar, ampule, package, vessel, bag, box, bin, envelope, carton, container, silo, shipping container, truck bed, and case.

In certain examples, the bacteria as taught herein are heterologous to the plant or plant part to be treated or administered with the same. A bacterium is considered heterologous to the plant, plant part, seed for growing the plant, or locus of the plant if the plant, plant part, seed for growing the plant, or locus of the plant that is untreated (e.g., a seed that is not treated with a bacterial strain described herein) does not contain detectable levels of the bacterium. A bacterium is considered "heterologously disposed" or "heterologous disposed" on the exterior surface of or within a plant or plant tissue when the bacterium is applied or disposed on the plant in a number that is not found on that plant before application of the bacterium. For example, a purified bacterial strain disposed on an exterior surface or within the seed can be an endophytic bacterium that may be associated with the mature plant, but is not found on the surface of or within the seed. As such, a bacterium is deemed heterologously disposed when applied on the plant that either does not naturally have the bacterium on its surface or within the particular tissue to which the bacterium is disposed, or does not naturally have the bacterium on its surface or within the particular tissue in the number that is being applied.

In certain examples, the strain is heterologous disposed, for example, on the surface of a reproductive element of a plant, in an amount effective to be detectable in the mature plant. In a particular example, the strain is heterologous disposed in an amount effective to be detectable in an amount of at least about 100 CFU, between 100 and 200 CFU, at least about 200 CFU, between 200 and 300 CFU, at least about 300 CFU, between 300 and 400 CFU, at least about 500 CFU, between 500 and 1,000 CFU, at least about 1,000 CFU, between 1,000 and 3,000 CFU, at least about 3,000 CFU, between 3,000 and 10,000 CFU, at least about 10,000 CFU, between 10,000 and 30,000 CFU, at least about 30,000 CFU, between 30,000 and 100,000 CFU, at least about 100,000 CFU or more in the mature plant.

In a preferred example, the bacteria are heterologously disposed to a plant, part thereof, seed for growing the plant, or locus of the plant in an amount effective to improve the plant growth feature, such as increase the biomass, height, and/or yield, of the treated plant relative to an untreated plant. In a preferred example, the amount of the heterologous disposed strain to the plant, part thereof, seed for growing the plant, or locus of the plant is effective to maintain a critical population mass in the plant. In a further example, the amount of the heterologous disposed strain to a seed for growing a plant is effective to maintain a critical population mass in the mature plant germinated from the seed. Hence, in certain examples, any of the bacterial strains, bacterial populations, or microbial active ingredients as contemplated herein may be heterologous disposed to the plant, plant part, or seed.

The above aspects and examples are further supported by the following non-limiting examples.

### EXAMPLES

### Example 1: Storage, cultivation, and formulation of bacteria according to certain examples of the disclosure

The bacterial strain deposited the BCCM^{™} / LMG Bacteria collection (BCCM^{™}/LMG) on 24 December 2021 under Accession No. LMG P-32513 (with the following identification reference given to the deposited material by the depositor: **BML-B-A11E4(2)**) (proposed taxonomic designation *Stenotrophomonas rhizophila*) (henceforth referred to in the Examples as "the A11E4 strain", "the bacterial strain", or "the strain") was stored at -75°C in nutrient broth (beef extract 0.3% w/v, peptone 0.5% w/v, 0.5% w/v sodium chloride, in distilled water, pH 6.8 ± 0.2) amended with 25% v/v glycerol and was cultured on nutrient agar (composition as nutrient broth with 1.5% w/v agar) for at least three days at 28°C. Cell suspensions were prepared by harvesting cells which have been incubated from single colonies in nutrient broth between two and five days in a shaker at 28°C degrees. Harvesting was done through centrifugation at 5000 g for 5 minutes. Alternatively, the whole cell broth, comprising the bacteria as well as the medium in which the bacteria can be used for inoculation.

Bacterial biomass was resuspended and resuspended in 1500 microliter 1% Carboxymethycelluose (w/v, dissolved in phosphate buffer) and added to 8.2 gram of wheat seeds for greenhouse (GH) testing (see Example 2).

Bacterial biomass was resuspended in a small volume (10 ml per 10 g of carrier) of nutrient broth and inoculated onto a carrier (peat) and mixed through shaking. The mix was incubated for 72 hours at room temperature. Per kg of seeds to be coated, 50 ml of a sticker solution in water containing 1% w/v methyl cellulose and 1% w/v colorant (Agrocer^{®} Red 112, Clariant International, Muttenz, Switzerland) together with the bacterial biomass on the carrier was added to the seeds. The final CFU per seed for the A11E4 bacterial strain was at least 1 x 10^3 cells per seed. Seeds were stored at 4°C before sowing. See Examples 3 and 4.

### Example 2: Increased dry biomass, wet biomass, and plant height per plant in wheat treated by a method according to certain examples of the disclosure

Per treatment, 5 x 24 wheat seeds were treated with a formulation containing a *Stenotrophomonas rhizophila* strain A11E4, as described in Example 1. Five planter boxes were filled with potting soil mix and saturated with water. As a control, 10 x 24 wheat seeds were treated with a formulation without bacterial strain to compare (mock treatment). Seeds were sown in three rows of 8 seeds per planter box. Nutrients were being added to the planter boxes at two and three weeks after sowing. The number of tillers per plant were counted 5 weeks after sowing from the wheat plants obtained from seeds treated with said bacterial strain. After 6 weeks of growth, shoots were cut off and fresh biomass (in g) was weighed per planter box (i.e., all 24 shoots together). Plant shoots were then dried at 60°C for 1 week and dry biomass (in g) was determined per planter box.

For all evaluated formulations, each containing a bacterial strain according to an example of the disclosure, an increase in dry biomass, wet biomass and an increase in plant height per plant was seen compared to the formulation without bacterial strain. The results of wheat treated with a formulation containing a *Stenotrophomonas rhizophila* strain A11E4 are shown in Figure 1.

The graphs visualize the values of different parameters with 95% confidence intervals for A11E4 treated seeds and mock treated seeds. The dashed line in the graphs (right) represents the mock treatment. The increase of 12.3% in dry biomass compared to a formulation without bacterial strain is visualized in Figure 1A. The increase of 11.4% in wet biomass compared to a formulation without bacterial strain is visualized in Figure 1B. The increase of 4.6% in plant height per wheat plant compared to a formulation without bacterial strain is visualized in Figure 1C.

### Example 3: Increased grain yield in wheat plants grown in the field from wheat seeds treated by a method according to an example of the disclosure

Per treatment, 1.5 kg winter or spring wheat seeds were coated with a formulation containing a *Stenotrophomonas rhizophila* A11E4 strain and a colorant as described in Example 1. Seeds were sown on 4 replicate plots (15 m² plot size) per field location using standard agricultural practices Sowing density was 300 to 400 seeds per m2. Sowing was done from mid to end of October for winter wheat and later March for spring wheat. Harvest happened for both around the end of July. Fertilization was calculated based on soil analysis. 50 kg of phosphorus (P2O5) and 50 kg of potassium (K2O) fertilizers were applied at sowing time. Nitrogen fertilizer was applied at 50 or 80 % level at two or three timings for respectively spring and winter wheat: 80 kg N ha-1 at tillering stage, 60 - 80 kg N ha-1 at 2-3 node stage and 40 kg N ha-1 at flag leaf stage (100 % nutrient level). Harvest was done with a Delta plot combine (Wintersteiger AG, Ried, Austria) and seed yield (grain yield) (kg/ha) was calculated based on the grain yield harvested at each individual plot and considering a seed moisture of 14 %. Grain yield was compared with untreated seeds. The results of the wheat plants grown from wheat seeds coated with a formulation containing a *Stenotrophomonas rhizophila* A11E4 strain are visualized in Figure 2-4.

Figure 2A visualizes the value in grain yield of spring wheat measured at one location with 95% confidence intervals for coated seeds and untreated seeds. The dashed line in the graphs represent the untreated seeds. Wheat treated with the formulation containing a *Stenotrophomonas rhizophila* A11E4 strain and colorant showed an increased yield of 13.5 % compared to the untreated seeds.

Figure 2B visualizes the value in grain yield of spring wheat measured at one location with 95% confidence intervals for coated seeds and untreated seeds. The dashed line in the graphs represent the untreated seeds. Wheat treated with the formulation containing a *Stenotrophomonas rhizophila* A11E4 strain and colorant showed an increased yield of 3.5 % compared to the untreated seeds.

Figure 3A visualizes the value in grain yield of winter wheat measured at one location with 95% confidence intervals for coated seeds and untreated seeds. The dashed line in the graphs represent the untreated seeds. Wheat treated with the formulation containing a *Stenotrophomonas rhizophila* A11E4 strain and colorant showed an increased yield of 8.6 % compared to the untreated seeds.

Figure 3B visualizes the value in grain yield of winter wheat measured at one location with 95% confidence intervals for coated seeds and untreated seeds. The dashed line in the graphs represent the untreated seeds. Wheat treated with the formulation containing a *Stenotrophomonas rhizophila* A11E4 strain and colorant showed an increased yield of 11.5 % compared to the untreated seeds.

Figure 4A visualizes the value in grain yield of winter wheat measured at one location with 95% confidence intervals for coated seeds and untreated seeds. The dashed line in the graphs represent the untreated seeds. Wheat treated with the formulation containing a *Stenotrophomonas rhizophila* A11E4 strain and colorant showed an increased yield of 15,2 % compared to the untreated seeds.

Figure 4B visualizes the value in grain yield of winter wheat measured at one location with 95% confidence intervals for coated seeds and untreated seeds. The dashed line in the graphs represent the untreated seeds. Wheat treated with the formulation containing a *Stenotrophomonas rhizophila* A11E4 strain and colorant showed an increased yield of 11.8 % compared to the untreated seeds.

### Example 4: Evidence for root endophytic behavior in the field of Stenotrophomonas rhizophila according to certain examples of the disclosure

Winter wheat plants originating from seeds coated with a formulation containing a *Stenotrophomonas rhizophila* A11E4 strain and a colorant, as described in Example 1; and seeds coated with a mock formulation mock formulation as described in Example 1, where harvested from the field four months after sowing in February. Five individual plants were collected for each treatment. Roots were washed using phosphate-buffered saline (PBS) to remove the rhizosphere soil attached to them and DNA was subsequently isolated from the roots. Microbiome mapping of the wheat root samples was performed by bacterial 16S rRNA gene amplicon sequencing using the primer pair Forward primer 27F: AGAGTTTGATCCTGGCTCAG (SEQ ID NO: 2); Reverse primer1492R: GGTTACCTTGTTACGACTT (SEQ ID NO: 3). The relative abundance of the amplicon corresponding to strain A11E4 (see SEQ ID NO: 1) in the wheat roots harvested from the field four months after sowing is shown in Figure 5 (as determined by next-generation sequencing).

### Example 5: Increased yield in wheat plants grown in the field from wheat seeds treated by a method according to an example of the disclosure

Per treatment, 1.5 kg winter wheat seeds were coated with a formulation containing a *Stenotrophomonas rhizophila* A11E4 strain and a colorant as described in Example 1. Seeds were sown on 4 replicate plots (12 to 18 m² plot size) per field location using standard agricultural practices. Sowing density was 360 to 400 seeds per m2. Sowing was done from the beginning of to mid October for winter wheat. Harvest happened around the end of July. Fertilization was calculated based on soil analysis. 50 kg of phosphorus (P2O5) and 50 kg of potassium (K2O) fertilizers were applied at sowing time. Nitrogen fertilizer was applied at 70 or 100 % level at two or three timings: 80 kg N ha-1 at tillering stage, 60 - 80 kg N ha-1 at 2-3 node stage and 40 kg N ha-1 at flag leaf stage (100 % nutrient level). Harvest was done with a Delta plot combine (Wintersteiger AG, Ried, Austria) and seed yield (grain yield) (kg/ha) was calculated based on the grain yield harvested at each individual plot and considering a seed moisture of 14 %. Grain yield was compared with untreated seeds. The results of the wheat plants grown from wheat seeds coated with a formulation containing a *Stenotrophomonas rhizophila* A11E4 strain are visualized in Figure 6.

Figure 6A visualizes the value in grain yield of winter wheat measured with 95% confidence intervals at a location in Poland in the season 2021-2022 with a 70 % N fertilizer regime. Wheat treated with the spray-dried formulation containing a *Stenotrophomonas A11E4 strain* and colorant showed an increased yield of 7.7 % compared to the untreated seeds.

Figure 6B visualizes the value in grain yield of winter wheat measured with 95% confidence intervals at a location in Bulgaria in the season 2021-2022 with a 70 % N fertilizer regime. Wheat treated with the spray-dried formulation containing a *Stenotrophomonas A11E4 strain* and colorant showed an increased yield of 7.3 % compared to the untreated seeds.

Figure 6C visualizes the value in grain yield of winter wheat measured with 95% confidence intervals at a location in Germany in the season 2021-2022 with a 100 % N fertilizer regime. Wheat treated with the spray-dried formulation containing a *Stenotrophomonas A11E4 strain* and colorant showed an increased yield of 2.1 % compared to the untreated seeds.

Figure 6D visualizes the value in grain yield of winter wheat measured with 95% confidence intervals at a location in Bulgaria in the season 2021-2022 with a 100 % N fertilizer regime. Wheat treated with the spray-dried formulation containing a *Stenotrophomonas A11E4 strain* and colorant showed an increased yield of 5.8 % compared to the untreated seeds.

### Example 6: Beneficial effects of bacterial cells according to an example of the disclosure on certain characteristics of plants

### Colonization of plants

Motility is considered as an important trait for a variety of biological functions such as wider access to nutrient sources, avoidance of toxic substances, ability to translocate to preferred locations, access to optimal colonization sites and biofilm formation. We assessed motility *in vitro* by using a Nutrient Agar (NA) semi solid medium according to Cousin et *al*. (Cousin et al. Appl Environ Microbiol. 2015, vol. 81, 1297-1308). The Falcon tubes were incubated at 28 °C for up to one week and checked daily. Negative controls containing no microbial strains and negative controls containing no motile strains (*in vitro* and *in silico* previously investigated) belonging to the Aphea.bio microbial collection were included. Results were qualitatively evaluated by observing microbial growth. Bacteria that are motile grow away from the area of inoculation, spreading towards the edges of the tube. The strain A11E4 was scored as positive in all the three replicates.

Biofilms are bacterial communities in which cells are embedded in a matrix of extracellular polymeric compounds attached to a surface. These living structures help protect bacteria from deleterious conditions and at the same time play an important role in root colonization. We assessed *in vitro* biofilm production spectrophotometrically in 96-well plates using an optimized protocol based on the dye red safranin (Allkja et al. Biofilm. 2020, vol. 2, 100010). This compound binds to cells and negatively-charged molecules (such as polysaccharides, main compounds in a microbial biofilm matrix). Negative controls containing no bacterial strains were included. The strain A11E4 was scored as positive after analyses at the spectrophotometer (emission peak at the wavelength of 535 nm).

Reactive oxygen species (ROS) are produced as a normal product of plant cellular metabolism. However, environmental stresses lead to excessive production of ROS causing progressive oxidative damage and ultimately cell death. Some microorganisms are capable of controlling ROS concentrations via enzymatic reactions. For instance, the activity of the catalase enzyme neutralizes the effects of hydrogen peroxide. Catalase expedites the breakdown of hydrogen peroxide (H₂O₂) into water and oxygen (2H₂O₂ + Catalase → 2H₂O + O₂). We assessed *in vitro* the presence (and activity) of the enzyme catalase according to Reiner K. 2010 (Catalase test protocol. American society of microbiology; https://asm.org/getattachment/72a871fc-ba92-4128-a194-6f1bab5c3ab7/Catalase-Test-Protocol.pdf).

When hydrogen peroxide is added to the microbial sample if the enzyme catalase is active we observe a rapid formation of bubbles. This reaction was not observed in the negative control (no catalase enzyme to hydrolyze the hydrogen peroxide). The strain A11E4 was qualitatively scored as positive.

We confirmed colonization of wheat rhizosphere soil and wheat roots by the A11E4 *Stenotrophomonas rhizophila* strain in samples collected from multiple field trials performed across Europe in different soil types and climatic zones (**Figure 7**). Winter wheat plants originating from seeds coated with a formulation containing the A11E4 strain and a colorant; and seeds coated with a mock formulation containing only colorant, where harvested from the field three to four months after sowing when the plants reached developmental stage BBCH16-17. Five individual plants were collected for each treatment. Roots with rhizosphere soil still attached to them were carefully removed from the soil and placed in phosphate buffered saline (PBS) to retrieve the rhizosphere soil after centrifugation (5 min at 2000 rpm). Roots were placed in fresh PBS buffer before centrifugation and washed with PBS to remove all soil, before storing the root samples at -80°C until DNA extraction. DNA was subsequently isolated from the roots and rhizosphere soil samples. Microbiome mapping of the samples was then performed by bacterial 16S rRNA gene amplicon sequencing of the V3-V4 region of the 16S rRNA gene. The relative abundance of the amplicon corresponding to the A11E4 strain compared to other 16S rRNA amplicons (as determined by next-generation sequencing) in the wheat rhizosphere soil and wheat root samples harvested from the field at growth developmental stage BBCH16-17 is shown in **Figure 7****.** Results are shown for six field trials performed across Europe and demonstrate that the A11E4 bacterial strain successfully colonizes wheat roots and/or wheat rhizosphere soil in the field after seed coating.

### P solubilization

Phosphate frequently limits plant growth due to its presence in soil as an insoluble form that cannot be used by plants.

We assessed and confirmed **phosphatase** enzyme activity (organic and/or inorganic P solubilization) by optimizing the use of the EnzChek^{™} Phosphatases Assay Kit (Invitrogen by Thermo Fisher Scientific) according to the manufacturer's instruction. The kit continuously detects phosphatase activity at neutral, alkaline, or acidic pH thanks to a patented molecular probe substrate (DiFMUP). The reaction product has excitation/emission maxima of 358/455 nm. Negative (blank samples not containing the A11E4 strain) and two positive controls (Type Strain DSM 17497 from DSMZ Collection of Microorganisms and acid phosphatases from potato) were included (**Figure 8**).

We investigated the total P content in shoots of wheat plants treated with the A11E4 bacterial strain. For this purpose, wheat seeds (variety Reform) were coated with sticker, colorant, and spray-dried product of the A11E4 strain at a rate of 5g/kg seeds (A11E4 treatment) or with sticker and colorant only (Mock treatment). For each treatment, five planter boxes containing substrate were set up, with each box containing 24 wheat seeds. Plants were maintained in the greenhouse and harvested 6 weeks after sowing. Shoots were dried, dry biomass was determined and samples sent for nutrient analysis. The dry biomass and total relative shoot P content (mg/kg dry material) are shown in **Figure 9****.** A significant biostimulant effect (increase of 10,6%, *p*=0.003) was observed for plants treated with the A11E4 bacterial strain, as is evident from the higher dry biomass compared to the Mock treatment (**Figure 9A**). Moreover, we detected a higher relative shoot P content in plants treated with the A11E4 strain (an average increase of 16% that was 18 times higher than the analytical error of 15 mg/kg P; **Figure 9B**), which clearly demonstrates that the A11E4 strain enhanced the availability of phosphate for plant uptake and thereby improved plant growth, resulting in a higher dry biomass at harvest.

### Iron availability

The ability to produce different siderophores can help increase iron availability for the plant. Iron is an important micronutrient for several vital processes in plants, such as respiration and photosynthesis. Through chelation, siderophores offer a high-affinity system for the uptake of iron from the environment.

We confirmed siderophore production by the A11E4 strain *in vitro.* We assessed siderophore production by using an optimized version of the Blue Agar CAS Assay (Louden et al. Journal of Microbiology &amp. 2011, vol. 12, 51-53) originally developed by Schwyn and Neiland. The experiment was carried out on agar plates, in triplicate. The universal siderophore assay is based on chrome azurol S (CAS) and hexadecyltrimethylammonium bromide (HDTMA) used as indicators. The CAS/HDTMA complexes tightly with ferric iron to produce a blue color. When a strong iron chelator such as a siderophore removes iron from the dye complex, the color changes from blue to orange. Negative (blank samples not containing the A11E4 strain) and positive controls (Type Strain DSM 14164 from DSMZ Collection of Microorganisms) were included in the assay. Due to the visible halo around its colonies the strain A11E4 was scored as positive.

### Osmoprotection

By producing osmolytes, such as trehalose and glucosylglycerol, bacterial cells can protect themselves from high salt concentrations and simultaneously protect plant roots from osmotic stress in the environment, ultimately resulting in a better growth of the plant.

Through whole genome analysis, we confirmed the potential of the A11E4 bacterial strain to produce the osmolytes trehalose (otsA, treY, and treZ genes, involved in trehalose biosynthesis) and glucosylglycerol (ggpS gene, involved in glucosylglycerol biosynthesis). Furthermore, the A11E4 strain also has the potential to produce exopolysaccharides (epsC gene, involved in EPS biosyntehsis), which among several other beneficial functions for plant-bacterium interactions can also be involved in osmoprotection.

### Development of a larger root system

Wheat plants treated with the A11E4 bacterial strain developed a larger root system compared to the Mock. This was evident *in vitro* after germination of wheat seeds on soil extract medium agar plates (**Figure 10**). For this purpose, wheat seeds (variety Reform) were incubated for 3 hours at 28°C whilst shaking in a solution containing either sticker plus a culture of bacterial cells of the A11E4 strain (A11E4 treatment), or sticker only (Mock treatment). Seeds (30 replicates per treatment) were subsequently placed on agar plates containing soil extract and incubated at room temperature in the dark. Seven days after inoculation, the wheat seedlings were carefully removed from the agar plates and the root systems were scanned. Image analysis was performed on the root pictures to determine both the total root surface area and the root circumference, using scripts developed in-house. Wheat plants treated with bacterial strain A11E4 showed a significantly higher root surface and root circumference compared to the Mock (**Figure 10**), demonstrating that strain No. B/00289 results in the development of a larger root system.

### Biostimulant effect on Arabidopsis thaliana

Bacterial strain A11E4 showed a significant biostimulant effect on the model plant *Arabidopsis thaliana,* demonstrating the robustness of its biostimulant properties. A growth promotion assay in *A. thaliana* was performed *in vitro.* For this purpose, the bacterial strain A11E4 was grown freshly before use on nutrient agar medium plates at 28 ⁰C. To create a bacterial inoculum, strains were grown overnight in a liquid culture of nutrient broth at 28 °C, diluted the next morning and again grown for 3-4 h to reach the exponential growth phase. The culture was then centrifuged at 2,500 rcf for 10 min and the bacterial pellet was resuspended and diluted to an optical density (OD₆₀₀) of 0.01 with phosphate buffered saline (PBS). Wild type *A. thaliana* Col0 seeds were surface sterilized with chlorine gas followed by stratification at 4°C in the dark for 2 days. Seeds were sown on agar-solidified plant growth medium (2.3 g/l MS, 0.5 g/l MES, 8 g/l plant tissue culture agar; pH 5.7) and incubated vertically in a tissue culture room at 21°C under long-day conditions (16 h light/8 h dark). Four-day-old seedlings of the same size were transferred to new plates (5 per plate, 3 plates per condition) and inoculated by pipetting 8 µl on the root tip of the bacterial inoculum (OD₆₀₀ = 0.01; B00289 treatment) or PBS in control conditions (Mock treatment) and further grown vertically in the tissue culture room. At 14 days post inoculation shoot and root fresh weight were determined, and the percentage of growth promotion was calculated. This experiment was executed three times. Inoculation with bacterial strain No. A11E4 resulted in significant increases in the total fresh weight of 62%, 59% and 49% vs. the Mock treatment, respectively, in the three independent experiments (results of the second experiment are shown in **Figure 11**). This clearly demonstrates the robustness of the biostimulant properties of bacterial strain No. A11E4 on other plant species, such as the model plant *A. thaliana.*

### DEPOSIT OF BIOLOGICAL MATERIAL

The purified bacterial strain (proposed taxonomic designation *Stenotrophomonas rhizophila*) as taught herein has been deposited at the BCCM^{™} / Laboratory of Microbiology (LMG) Bacteria collection (BCCM^{™}/LMG), on 24 December 2021 under Accession No. LMG P-32513 (with the following identification reference given to the deposited material by the depositor: **BML-B-A11E4(2)**). Table 1 summarizes the requisite indications relating to this deposited microorganism. For reasons of brevity, this strain is also referred to as strain "BML-B-A11E4(2)" or strain "A11E4" throughout this specification.

**Table 1**

| | |
|---|---|
| Accession number given by depositary institution | LMG P-32513 |
| Identification reference given by the depositor | BML-B-A11E4(2) |
| Name of depositary institution | Belgian Coordinated Collections of Microorganisms (BCCM^{™}) / LMG Bacteria collection (BCCM^{™}/LMG) |
| Address of depositary institution | Universiteit Gent |
| | Laboratorium voor Microbiologie (LMG) |
| | K.L. Ledeganckstraat 35 |
| | 9000 Gent |
| | Belgium |
| Date of deposit | 24 December 2021 |
| Depositor | Aphea.Bio NV |
| Address of depositor | Technologiepark 21, B-9052 Zwijnaarde, Belgium |
| Proposed taxonomic designation | *Stenotrophomonas rhizophila* |

### SEQUENCE LISTING

Throughout the description and examples, reference is made to the following sequences, as shown in Table 2:
SEQ ID NO: 1: Nucleotide sequence of 16S polynucleotide from strain as deposited under the Budapest Treaty at the BCCM^{™} / Laboratory of Microbiology (LMG) Bacteria collection (BCCM^{™}/LMG), on 24 December 2021 with the following identification reference given to the deposited microorganism by the depositor: **BML-B-A11E4(2),** and the accession number LMG P-32513 given by the depositary authority.

**Table 2**

| **Strain** | **SEQ ID NO** | **Sequence of 16S polynucleotide (5' - 3')** |
|---|---|---|
| **BML-B-A11E4(2)** | **1** | GTGAAGAGTTTGATCCTGGCTCAGAGTGAACGCTGGCGGTAGGCCTAACA CATGCAAGTCGAACGGCAGCACAGTAAGAGCTTGCTCTTATGGGTGGCGA GTGGCGGACGGGTGAGGAATACATCGGAATCTACCTTTTCGTGGGGGATA ACGTAGGGAAACTTACGCTAATACCGCATACGACCTTCGGGTGAAAGCAG GGGACCTTCGGGCCTTGCGCGGATAGATGAGCCGATGTCGGATTAGCTAG TTGGCGGGGTAAAGGCCCACCAAGGCGACGATCCGTAGCTGGTCTGAGAG GATGATCAGCCACACTGGAACTGAGACACGGTCCAGACTCCTACGGGAGG CAGCAGTGGGGAATATTGGACAATGGGCGCAAGCCTGATCCAGCCATACC GCGTGGGTGAAGAAGGCCTTCGGGTTGTAAAGCCCTTTTGTTGGGAAAGA AAAGCAGTCGGCTAATACCCGGTTGTTCTGACGGTACCCAAAGAATAAGC ACCGGCTAACTTCGTGCCAGCAGCCGCGGTAATACGAAGGGTGCAAGCGT TACTCGGAATTACTGGGCGTAAAGCGTGCGTAGGTGGTTGTTTAAGTCTGT TGTGAAAGCCCTGGGCTCAACCTGGGAATTGCAGTGGATACTGGGCGACT AGAGTGTGGTAGAGGGTAGTGGAATTCCCGGTGTAGCAGTGAAATGCGTA GAGATCGGGAGGAACATCCATGGCGAAGGCAGCTACCTGGACCAACACTG ACACTGAGGCACGAAAGCGTGGGGAGCAAACAGGATTAGATACCCTGGT AGTCCACGCCCTAAACGATGCGAACTGGATGTTGGGTGCAATTTGGCACG CAGTATCGAAGCTAACGCGTTAAGTTCGCCGCCTGGGGAGTACGGTCGCA AGACTGAAACTCAAAGGAATTGACGGGGGCCCGCACAAGCGGTGGAGTA TGTGGTTTAATTCGATGCAACGCGAAGAACCTTACCTGGTCTTGACATGTC GAGAACTTTCCAGAGATGGATTGGTGCCTTCGGGAACTCGAACACAGGTG CTGCATGGCTGTCGTCAGCTCGTGTCGTGAGATGTTGGGTTAAGTCCCGCA ACGAGCGCAACCCTTGTCCTTAGTTGCCAGCACGTAATGGTGGGAACTCTA AGGAGACCGCCGGTGACAAACCGGAGGAAGGTGGGGATGACGTCAAGTC ATCATGGCCCTTACGACCAGGGCTACACACGTACTACAATGGTAGGGACA GAGGGCTGCAAACCCGCGAGGGCAAGCCAATCCCAGAAACCCTATCTCAG TCCGGATTGGAGTCTGCAACTCGACTCCATGAAGTCGGAATCGCTAGTAAT CGCAGATCAGCATTGCTGCGGTGAATACGTTCCCGGGCCTTGTACACACCG CCCGTCACACCATGGGAGTTTGTTGCACCAGAAGCAGGTAGCTTAACCTTC GGGAGGGCGCTTGCCACGGTGTGGCCGATGACTGGGGTGAAGTCGTAAC AAGGTAGCCGTATCGGAAGGTGCGGCTGGATCACCTCCTTT |

## Claims

1. A method for improving a plant growth feature of a plant compared to an untreated plant, the method comprising administering bacteria of a bacterial strain to the plant, a part thereof, a seed for growing the plant, or a locus of the plant, wherein the bacterial strain is the strain as deposited under the Budapest Treaty at the Belgian Coordinated Collections of Microorganisms (BCCM^{™}) / LMG Bacteria collection (BCCM^{™}/LMG) on 24 December 2021 under Accession No. LMG P-32513.

2. The method according to claim 1, comprising administering to the plant, the part thereof, the seed for growing the plant, or the locus of the plant the bacterial strain as defined in claim 1 in conjunction with one or more additional plant-beneficial microorganism.

3. The method according to any one of claims 1 to 2, wherein the bacteria are administered in an agricultural active composition, such as wherein:
- the composition further comprises one or more agriculturally acceptable auxiliary, such as a solvent, a carrier, a surfactant, a sticker, an antifreeze agent, a thickener, a buffering agent, an antifoaming agent, an antioxidant, a preservative, an aroma, a colorant, or a combination thereof;
- the composition is a liquid composition, such as an aqueous composition, such as a sprayable liquid or a concentrate, preferably wherein the composition comprises the bacteria at a concentration of at least 10² CFU/ml; or
- the composition is a non-liquid composition, such as a powder, preferably wherein the composition comprises the bacteria at an amount of at least 10² CFU/g.

4. The method according to any one of claims 1 to 3, wherein the method comprises:
- administering the bacteria or the composition to a seed of the plant, a whole plant, or a seedling, such as to a seed prior to planting the seed, or with the seed at planting, or after planting the seed and before germination of the seed, and optionally cultivating the seed, whole plant, or seedling under conditions to promote plant growth and development; or
- inoculating soil or a plant growth medium with the bacteria and growing the plant, such as from a seed, in said soil or medium.

5. A method of treating a seed of a plant comprising heterologously disposing the bacterial strain as defined in claim 1 to the seed , optionally in conjunction with one or more additional plant-beneficial microorganism, such that the bacteria colonize a plant germinated from the inoculated seed and/or the soil or plant growth medium surrounding the growing plant, whereby a plant growth feature of the plant, such as the biomass, height, and/or yield of the plant, is improved compared to a plant germinated from an untreated seed, optionally wherein the seed is coated with the bacteria, incubated with the bacteria, or planted near the bacteria.

6. A bacterial strain as deposited under the Budapest Treaty at the Belgian Coordinated Collections of Microorganisms (BCCM^{™}) / LMG Bacteria collection (BCCM^{™}/LMG) on 24 December 2021 under Accession No. LMG P-32513.

7. A bacterial population comprising the strain as defined in claim 6; or an agricultural active composition comprising the strain as defined in claim 6, optionally wherein:
- the composition further comprises one or more agriculturally acceptable auxiliary, such as a solvent, a carrier, a surfactant, a sticker, an antifreeze agent, a thickener, a buffering agent, an antifoaming agent, an antioxidant, a preservative, an aroma, a colorant, or a combination thereof;
- the composition is a liquid composition, such as an aqueous composition, such as a sprayable liquid or a concentrate, preferably wherein the composition comprises the bacteria at a concentration of at least 10² CFU/ml; or
- the composition is a non-liquid composition, such as a powder, preferably wherein the composition comprises the bacteria at an amount of at least 10² CFU/g.

8. A plant seed heterologously disposed on its surface with at least 10 CFU of the bacterial strain as defined in claim 6.

9. The method according to any one of claims 1 to 5, or the plant seed according to claim 8, wherein the plant is a monocotyledon, preferably wherein the plant is a cereal, more preferably wherein the plant is selected from the group consisting of wheat, maize, barley, rice, millet, rye, triticale, sorghum, emmer, spelt, einkorn, teff, milo, and oats, even more preferably wherein the plant is wheat or maize, such as wherein the plant is wheat.

10. The method according to any one of claims 1 to 5, or the plant seed according to claim 8, wherein the plant is a dicotyledon.

## Patentansprüche

1. Verfahren zur Verbesserung eines Pflanzenwachstumsmerkmals einer Pflanze im Vergleich zu einer unbehandelten Pflanze, wobei das Verfahren umfasst, das Verabreichen von Bakterien eines Bakterienstamms an die Pflanze, einen Teil davon, einen Samen zum Anbauen der Pflanze oder einen Standort der Pflanze umfasst, wobei der Bakterienstamm der Stamm ist, wie er gemäß dem Budapester Vertrag bei der Belgian Coordinated Collections of Microorganisms (BCCM^{™}) /LMG Bacteria collection (BCCM^{™}/LMG) am 24. Dezember 2021 unter der Depotnummer LMG P-32513 hinterlegt wurde.

2. Verfahren nach Anspruch 1, umfassend das Verabreichen des in Anspruch 1 definierten Bakterienstamms an die Pflanze, den Teil der Pflanze, den Samen zum Anbauen der Pflanze oder den Standort der Pflanze, in Verbindung mit einem oder mehreren zusätzlichen pflanzenfördernden Mikroorganismen.

3. Verfahren nach einem der Ansprüche 1 bis 2, wobei die Bakterien in einer landwirtschaftlich aktiven Zusammensetzung verabreicht werden, wie beispielsweise wobei:
die Zusammensetzung ferner ein oder mehrere landwirtschaftlich verträgliche Hilfsmittel umfasst, wie beispielsweise ein Lösungsmittel, einen Träger, ein Netzmittel, ein Haftmittel, ein Frostschutzmittel, ein Verdickungsmittel, ein Puffermittel, ein Antischaummittel, ein Antioxidationsmittel, ein Konservierungsmittel, ein Aroma, einen Farbstoff oder eine Kombination davon;
die Zusammensetzung eine flüssige Zusammensetzung ist, wie beispielsweise eine wässrige Zusammensetzung, wie beispielsweise eine sprühbare Flüssigkeit oder ein Konzentrat, wobei die Zusammensetzung vorzugsweise die Bakterien in einer Konzentration von mindestens 10² KBE/ml umfasst; oder
die Zusammensetzung eine nicht flüssige Zusammensetzung wie beispielsweise ein Pulver ist, wobei die Zusammensetzung vorzugsweise die Bakterien in einer Menge von mindestens 10² KBE/g umfasst.

4. Verfahren nach einem der Ansprüche 1 bis 3, wobei das Verfahren umfasst:
Verabreichen der Bakterien oder der Zusammensetzung an einen Samen der Pflanze, eine ganze Pflanze oder einen Sämling, wie beispielsweise an einen Samen vor dem Einpflanzen des Samens oder mit dem Samen beim Einpflanzen oder nach dem Einpflanzen des Samens und vor der Keimung des Samens, und optional das Kultivieren des Samens, der ganzen Pflanze oder des Sämlings unter Bedingungen, die das Pflanzenwachstum und die Entwicklung fördern; oder
Inokulieren von Boden oder einem Pflanzenwachstumsmedium mit den Bakterien und Anbauen der Pflanze, wie beispielsweise aus einem Samen, in dem Boden oder Medium.

5. Verfahren zur Behandlung eines Samens einer Pflanze, umfassend ein heterologes Einbringen des in Anspruch 1 definierten Bakterienstamms in den Samen, optional in Verbindung mit einem oder mehreren zusätzlichen pflanzenfördernden Mikroorganismen derart, dass die Bakterien eine aus dem inokulierten Samen gekeimte Pflanze und/oder den Boden oder das Pflanzenwachstumsmedium, das die wachsende Pflanze umgibt, besiedeln,
wodurch ein Pflanzenwachstumsmerkmal der Pflanze, wie beispielsweise die Biomasse, die Höhe und/oder der Ertrag der Pflanze, im Vergleich zu einer aus einem unbehandelten Samen gekeimten Pflanze verbessert wird, wobei der Samen optional mit den Bakterien beschichtet, mit den Bakterien inkubiert oder in der Nähe der Bakterien eingepflanzt wird.

6. Bakterienstamm, wie er gemäß dem Budapester Vertrag bei der Belgian Coordinated Collections of Microorganisms (BCCM^{™}) /LMG Bacteria collection (BCCM^{™}/LMG) am 24. Dezember 2021 unter der Depotnummer LMG P-32513 hinterlegt wurde.

7. Bakterienpopulation, die den in Anspruch 6 definierten Stamm umfasst; oder eine landwirtschaftlich aktive Zusammensetzung, die den in Anspruch 6 definierten Stamm umfasst, optional, wobei:
die Zusammensetzung ferner ein oder mehrere landwirtschaftlich verträgliche Hilfsmittel umfasst, wie beispielsweise ein Lösungsmittel, einen Träger, ein Netzmittel, ein Haftmittel, ein Frostschutzmittel, ein Verdickungsmittel, ein Puffermittel, ein Antischaummittel, ein Antioxidationsmittel, ein Konservierungsmittel, ein Aroma, einen Farbstoff oder eine Kombination davon;
die Zusammensetzung eine flüssige Zusammensetzung ist, wie beispielsweise eine wässrige Zusammensetzung, wie beispielsweise eine sprühbare Flüssigkeit oder ein Konzentrat, wobei die Zusammensetzung vorzugsweise die Bakterien in einer Konzentration von mindestens 10² KBE/ml umfasst; oder
die Zusammensetzung eine nicht flüssige Zusammensetzung wie beispielsweise ein Pulver ist, wobei die Zusammensetzung vorzugsweise die Bakterien in einer Menge von mindestens 10² KBE/g umfasst.

8. Pflanzensamen, auf dessen Oberfläche mindestens 10 KBE des in Anspruch 6 definierten Bakterienstammes heterolog angeordnet sind.

9. Verfahren nach einem der Ansprüche 1 bis 5 oder Pflanzensamen nach Anspruch 8, wobei die Pflanze eine Monokotyledone ist, vorzugsweise wobei die Pflanze ein Getreide ist, noch bevorzugter wobei die Pflanze aus der Gruppe bestehend aus Weizen, Mais, Gerste, Reis, Hirse, Roggen, Triticale, Sorghum, Emmer, Dinkel, Einkorn, Teff, Milo und Hafer ausgewählt ist, noch bevorzugter wobei die Pflanze Weizen oder Mais ist, insbesondere wobei die Pflanze Weizen ist.

10. Verfahren nach einem der Ansprüche 1 bis 5 oder Pflanzensamen nach Anspruch 8, wobei die Pflanze eine Dikotyledone ist.

## Revendications

1. Procédé pour améliorer une caractéristique de croissance végétale d'une plante par rapport à une plante non traitée, le procédé comprenant l'administration à la plante de bactéries d'une souche bactérienne, à une partie de celle-ci, à une graine pour la croissance de la plante ou à un locus de la plante, dans lequel la souche bactérienne est la souche déposée en vertu du Traité de Budapest auprès des Collections coordonnées belges de micro-organismes (BCCM^{™})/Collection de bactéries LMG (BCCM^{™}/LMG) le 24 décembre 2021 sous le numéro d'accès LMG P-32513.

2. Procédé selon la revendication 1, comprenant l'administration à la plante, à la partie de celle-ci, à la graine pour la croissance de la plante ou au locus de la plante de la souche bactérienne telle que définie dans la revendication 1 en conjonction avec un ou plusieurs micro-organismes supplémentaires bénéfiques pour la plante.

3. Procédé selon l'une quelconque des revendications 1 et 2, dans lequel les bactéries sont administrées dans une composition active agricole, telle que dans lequel :
- la composition comprend également un ou plusieurs auxiliaires acceptables en agriculture, tels qu'un solvant, un support, un tensioactif, un autocollant, un agent antigel, un épaississant, un agent tampon, un agent antimousse, un antioxydant, un conservateur, un arôme, un colorant ou une combinaison de ceuxci ;
- la composition est une composition liquide, telle qu'une composition aqueuse, telle qu'un liquide pulvérisable ou un concentré, de préférence dans lequel la composition comprend les bactéries à une concentration d'au moins 10² UFC/ml ; ou
- la composition est une composition non liquide, telle qu'une poudre, de préférence dans lequel la composition comprend les bactéries à une quantité d'au moins 10² UFC/g.

4. Procédé selon l'une quelconque des revendications 1 à 3, dans lequel le procédé comprend :
- l'administration des bactéries ou de la composition à une graine de la plante, à une plante entière ou à une plantule, par exemple à une graine avant de planter la graine, ou avec la graine lors de la plantation, ou après la plantation de la graine et avant la germination de la graine, et éventuellement la culture de la graine, la plante entière ou la plantule dans des conditions favorisant la croissance et le développement végétal ; ou
- l'inoculation du sol ou d'un milieu de croissance végétale avec les bactéries et la croissance de la plante, par exemple à partir d'une graine, dans ledit sol ou milieu.

5. Procédé de traitement d'une graine d'une plante, comprenant la disposition de manière hétérologue de la souche bactérienne telle que définie dans la revendication 1 dans la graine, éventuellement en association avec un ou plusieurs micro-organismes supplémentaires bénéfiques pour la plante, de sorte que les bactéries colonisent une plante germée à partir de la graine inoculée et/ou du sol ou du milieu de croissance végétale entourant la plante en croissance,
moyennant quoi une caractéristique de croissance végétale, telle que la biomasse, la hauteur et/ou le rendement de la plante, est améliorée par rapport à une plante germée à partir d'une graine non traitée, éventuellement dans lequel la graine est enrobée avec les bactéries, incubée avec les bactéries ou plantée à proximité des bactéries.

6. Souche bactérienne déposée en vertu du Traité de Budapest auprès des Collections coordonnées belges de micro-organismes (BCCM^{™}) /Collection de bactéries LMG (BCCM^{™}/LMG) le 24 décembre 2021 sous le numéro d'accès LMG P-32513.

7. Population bactérienne comprenant la souche telle que définie dans la revendication 6 ; ou composition active agricole comprenant la souche telle que définie dans la revendication 6, éventuellement dans laquelle :
- la composition comprend également un ou plusieurs auxiliaires acceptables en agriculture, tels qu'un solvant, un support, un tensioactif, un autocollant, un agent antigel, un épaississant, un agent tampon, un agent antimousse, un antioxydant, un conservateur, un arôme, un colorant ou une combinaison de ceuxci ;
- la composition est une composition liquide, telle qu'une composition aqueuse, telle qu'un liquide pulvérisable ou un concentré, de préférence dans laquelle la composition comprend les bactéries à une concentration d'au moins 10² UFC/ml ; ou
- la composition est une composition non liquide, telle qu'une poudre, de préférence dans laquelle la composition comprend les bactéries à une quantité d'au moins 10² UFC/g.

8. Graine de plante disposée de manière hétérologue sur sa surface avec au moins 10 UFC de la souche bactérienne telle que définie dans la revendication 6.

9. Procédé selon l'une quelconque des revendications 1 à 5, ou graine de plante selon la revendication 8, dans lequel ou laquelle la plante est une monocotylédone, de préférence dans lequel ou laquelle la plante est une céréale, de préférence dans lequel ou laquelle la plante est choisie dans le groupe constitué du blé, du maïs, de l'orge, du riz, du millet, du seigle, du triticale, du sorgho, de l'amidonnier, de l'épeautre, de l'engrain, du teff, du milo et de l'avoine, de préférence dans lequel ou laquelle la plante est le blé ou le maïs, et plus particulièrement dans lequel ou laquelle la plante est le blé.

10. Procédé selon l'une quelconque des revendications 1 à 5, ou graine de plante selon la revendication 8, dans lequel ou laquelle la plante est une dicotylédone.
